# EUROPEAN PATENT SPECIFICATION

(11) **EP 0 497 564 B1**
(45) Date of publication and mention of the grant of the patent: **27.12.1995**
(21) Application number: 92300726.4
(22) Date of filing: 28.01.1992
(51) Int. Cl.: C07C 235/36, A61K 31/165, C07C 311/39, A61K 31/18, C07C 321/30, C07C 255/60, C07C 327/48, C07D 213/75, A61K 31/44

(54) **Benzamides**
Benzamide
Benzamides

(30) Priority: 28.01.1991 GB 9101777; 16.08.1991 GB 9117727
(43) Date of publication of application: 05.08.1992
(60) Divisional application: 95106899.8
(73) Proprietor: RHONE-POULENC RORER LIMITED, West Malling, Kent ME19 4TA (GB)
(72) Inventor: Ashton, Michael John, Dagenham, Essex RM10 7XS (GB); Cook, David Charles, Dagenham, Essex RM10 7XS (GB); Fenton, Garry, Dagenham, Essex RM10 7XS (GB); Hills Susan Jacqueline, Dagenham, Essex RM10 7XS (GB); McFarlane, Ian Michael, Dagenham, Essex RM10 7XS (GB); Palfreyman, Malcolm Norman, Dagenham, Essex RM10 7XS (GB); Ratcliffe, Andrew James, Dagenham, Essex RM10 7XS (GB); Vicker, Nigel, Dagenham, Essex RM10 7XS (GB)
(74) Representative: Pilard, Jacques

(56) References cited:
- JP-A-62 158 252
- JOURNAL OF MEDICINAL CHEMISTRY, vol. 16, no. 4, April 1973, Washington, US, pages 332-336; I. W. MATHISON et al, "Synthesis and hypotensive properties of tetrahydroisoquinolines"
- CHEMICAL ABSTRACTS, vol. 108, no. 15, 11 April 1988, Columbus, Ohio, US; abstract no. 131583P, page 733, "Preparation of N-pyridyl-4-(benzyloxy)benzamides as cardiotonics"
- CHEMICAL ABSTRACTS, vol. 99, no. 6, 8 August 1983, Columbus, Ohio, US; abstract no. 43558z, page 322, "Hypoglycemic pharmaceuticals containing benzamide derivatives"
- BULLETIN DE LA SOCIETE CHIMIQUE DE FRANCE, 1965, Paris, FR, pages 848-857; P. GRAMMATICAKIS, "Contirbution à l'étude de l'absorption dans l'ultraviolet moyen et le visible des N-aroyl-arylamines. IV. 2,3-, 3,4- et 2,4-diméthoxybenzoylarylamines"

## Description

This invention relates to therapeutically useful benzamide derivatives, to processes for their preparation, to pharmaceutical compositions containing them, and to methods for their use.

The present invention provides compounds of general formula I, hereinafter depicted, wherein R¹ represents a straight- or branched-chain alkyl group containing up to about 4 carbon atoms, R² represents a straight- or branched-chain alkyl group containing from about 2 to about 15, preferably 2 to 12, carbon atoms or a mono-, bi- or tricycloalkyl group containing up to about 10 carbon atoms, R³ represents an optionally substituted phenyl, naphthyl or heterocyclyl group, preferably a 5-, 6- or 7-membered heterocyclyl group containing one or more hetero atoms selected from oxygen, sulphur and nitrogen atoms, the optional substituents being one or more substituents selected from halogen atoms, alkyl groups which may carry one or more halogen atoms, and from aryl, arylalkyl, alkoxy, aryloxy, alkylthio, arylthio, alkoxycarbonyl, aryloxycarbonyl, alkanoyl, aroyl, alkylsulphonyl, arylsulphonyl, alkylsulphinyl, arylsulphinyl, hydroxy, hydroxyalkyl, formyl, alkanoylamino, aroylamino, cyano and nitro groups, and from amino, carbamoyl and sulphamoyl groups which themselves may each carry one or two alkyl substituents, and Z represents an oxygen or sulphur atom, and when said heterocyclyl groups contain one or more nitrogen ring atoms, N-oxides thereof, and pharmaceutically acceptable salts thereof, wherein all aryl groups and moieties, unless otherwise indicated, are selected from phenyl and naphthyl groups optionally substituted by one or more substituents selected from halogen atoms and alkyl and alkoxy groups, and wherein all alkyl groups and moieties, unless otherwise indicated, are straight- or branched-chain and contain up to about 4 carbon atoms.

Especially important compounds of the invention include those wherein at least one of the symbols has a value selected from the following:-
(i) R¹ represents a methyl group;
(ii) R² represents a propyl, butyl, nonyl, dodecyl, cyclohexyl, 8,9,10-trinorbornyl or, more especially, cyclopentyl group; and/or
(iii) R³ represents an optionally substituted pyrazinyl, pyrimidinyl, isoxazolyl, preferably pyridyl group, or an N-oxide thereof, or an optionally substituted phenyl group;
   the other symbols being as hereinbefore defined.

Among compounds wherein R³ represents a substituted phenyl group, compounds wherein said phenyl group is substituted in the 2-position or in the 2- and 6-positions are especially useful.

Similarly, among compounds wherein R³ represents a substituted heterocyclyl group, compounds wherein said heterocyclyl group is substituted on one or both of the positions next to the point of attachment to the rest of the molecule are especially useful.

Individual compounds of especial importance include the following:-
- A: N-(2,6-difluorophenyl)-3-cyclopentyloxy-4-methoxybenzamide
- B: N-(2-chloro-6-fluorophenyl)-3-cyclopentyloxy-4-methoxybenzamide
- C: N-(2-trifluoromethylphenyl)-3-cyclopentyloxy-4-methoxybenzamide
- D: N-(2,4,6-trichlorophenyl)-3-cyclopentyloxy-4-methoxybenzamide
- E: N-(2,6-dibromophenyl)-3-cyclopentyloxy-4-methoxybenzamide
- F: N-(2-chloro-6-methylphenyl)-3-cyclopentyloxy-4-methoxybenzamide
- G: N-(2,6-dichlorophenyl)-3-cyclopentyloxy-4-methoxybenzamide
- H: N-(2-fluorophenyl)-3-cyclopentyloxy-4-methoxybenzamide
- I: N-phenyl-3-cyclopentyloxy-4-methoxybenzamide,
- J: N-(2-methoxyphenyl)-3-cyclopentyloxy-4-methoxybenzamide
- K: N-(2-chlorophenyl)-3-cyclopentyloxy-4-methoxybenzamide
- L: N-(3-chlorophenyl)-3-cyclopentyloxy-4-methoxybenzamide
- M: N-(4-methoxyphenyl)-3-cyclopentyloxy-4-methoxybenzamide
- N: N-(2,6-dimethylphenyl)-3-cyclopentyloxy-4-methoxybenzamide
- O: N-(2-methylthiophenyl)-3-cyclopentyloxy-4-methoxybenzamide
- P: N-(2-bromophenyl)-3-cyclopentyloxy-4-methoxy-benzamide
- Q: N-(2-methoxycarbonylphenyl)-3-cyclopentyloxy-4-methoxybenzamide
- R: N-(2-aminosulphonylphenyl)-3-cyclopentyloxy-4-methoxybenzamide
- S: N-(2-benzoylphenyl)-3-cyclopentyloxy-4-methoxybenzamide
- T: N-(2-cyanophenyl)-3-cyclopentyloxy-4-methoxybenzamide
- U: N-(2,5-dichlorophenyl)-3-cyclopentyloxy-4-methoxybenzamide
- V: N-(3-methylphenyl)-3-cyclopentyloxy-4-methoxybenzamide
- W: N-(2-nitrophenyl)-3-cyclopentyloxy-4-methoxybenzamide
- X: N-(2-dimethylaminophenyl)-3-cyclopentyloxy-4-methoxybenzamide
- Y: N-(2-acetylphenyl)-3-cyclopentyloxy-4-methoxybenzamide
- Z: N-(2-hydroxyphenyl)-3-cyclopentyloxy-4-methoxybenzamide
- AA: N-(2-methylsulphonylphenyl)-3-cyclopentyloxy-4-methoxybenzamide
- AB: N-(2,6-difluorophenyl)-3-cyclohexyloxy-4-methoxybenzamide
- AC: N-(2,6-difluorophenyl)-3-butoxy-4-methoxybenzamide
- AD: N-(2,6-difluorophenyl)-3-propoxy-4-methoxybenzamide
- AE: N-(2-chlorophenyl)-3-cyclopentyloxy-4-methoxy(thiobenzamide)
- AF: N-(4-chloropyrid-3-yl)-3-cyclopentyloxy-4-methoxybenzamide
- AG: N-pyrid-2-yl-3-cyclopentyloxy-4-methoxybenzamide
- AH: N-pyrazin-2-yl-3-cyclopentyloxy-4-methoxybenzamide
- AI: N-pyrimidin-2-yl-3-cyclopentyloxy-4-methoxybenzamide
- AJ: N-(3-methylpyrid-2-yl)-3-cyclopentyloxy-4-methoxybenzamide
- AK: N-pyrid-3-yl-3-cyclopentyloxy-4-methoxybenzamide
- AL: N-(3-chloropyrid-2-yl)-3-cyclopentyloxy-4-methoxybenzamide
- AM: N-(3-chloropyrid-4-yl)-3-cyclopentyloxy-4-methoxybenzamide
- AN: N-pyrid-4-yl-3-cyclopentyloxy-4-methoxybenzamide
- AO: N-(3,5-dichloropyrid-4-yl)-3-cyclopentyloxy-4-methoxybenzamide
- AP: N-(3,5-dimethylisoxazol-4-yl)-3-cyclopentyloxy-4-methoxybenzamide
- AQ: N-(4,6-dichloropyrimid-5-yl)-3-cyclopentyloxy-4-methoxybenzamide
- AR: N-(4-nitrophenyl)-3-cyclopentyloxy-4-methoxybenzamide
- AS: N-(2,3,5,6-tetrafluoropyrid-4-yl)-3-cyclopentyloxy-4-methoxybenzamide
- AT: N-(3,5-dichloro-2,6-difluoropyrid-4-yl)-3-cyclopentyloxy-4-methoxybenzamide
- AU: N-(2,4,6-trifluorophenyl)-3-cyclopentyloxy-4-methoxybenzamide
- AV: 3,5-dichloro-4-(3-cyclopentyloxy-4-methoxybenzamido)pyridine-N-oxide
- AW: N-(3,5-dichloropyrid-4-yl)-3-(exo-8,9,10-trinorbornyl-2-oxy)-4-methoxybenzamide
- AX: N-(3,5-dichloropyrid-4-yl)-3-cyclohexyloxy-4-methoxybenzamide
- AY: N-(3,5-dibromopyrid-4-yl)-3-cyclopentyloxy-4-methoxybenzamide
- AZ: N-(3,5-dichloropyrid-4-yl)-3-butoxy-4-methoxybenzamide
- BA: N-(3-methyl-5-bromoisothiazol-4-yl)-3-cyclopentyloxy-4-methoxybenzamide
- BB: N-(3,5-dimethylisothiazol-4-yl)-3-cyclopentyloxy-4-methoxybenzamide
- BC: N-(3,5-dimethylpyrid-4-yl)-3-cyclopentyloxy-4-methoxybenzamide
- BD: N-(5-cyano-3-methylisothiazol-4-yl)-3-cyclopentyloxy-4-methoxybenzamide
- BE: N-(3,5-dichloropyrid-4-yl)-3-cyclopentyloxy-4-methoxy(thiobenzamide)
- BF: N-(2,6-dichloro-4-methoxyphenyl)-3-cyclopentyloxy-4-methoxybenzamide
- BG: N-(2,6-dichloro-4-cyanophenyl)-3-cyclopentyloxy-4-methoxybenzamide
- BH: N-(2,6-dichloro-4-carbamoylphenyl)-3-cyclopentyloxy-4-methoxybenzamide
- BI: N-(2,6-dichloro-4-aminophenyl)-3-cyclopentyloxy-4-methoxybenzamide
- BJ: N-(3-chloro-2,5,6-trifluoropyrid-4-yl)-3-cyclopentyloxy-4-methoxybenzamide
- BK: N-(3,5-dibromopyrid-4-yl)-3-butoxy-4-methoxybenzamide
- BL: N-(2,6-dichloro-4-methoxycarbonylphenyl)-3-cyclopentyloxy-4-methoxybenzamide
- BM: N-(4-acetylamino-2,6-dichlorophenyl)-3-cyclopentyloxy-4-methoxybenzamide
- BN: N-(3,5-dichloropyrid-4-yl)-3-nonyloxy-4-methoxybenzamide
- BO: N-(2,6-dichloro-4-formylphenyl)-3-cyclopentyloxy-4-methoxybenzamide
- BP: N-(2,6-dichlorophenyl)-3-(exo-8,9,10-trinorbornyl-2-oxy)-4-methoxybenzamide
- BQ: N-(2,3,5-trifluoropyrid-4-yl)-3-cyclopentyloxy-4-methoxybenzamide
- BR: sodium salt of N-(3,5-dichloropyrid-4-yl)-3-cyclopentyloxy-4-methoxybenzamide
- BS: N-(2,6-dichloro-4-ethoxycarbonylphenyl)-3-cyclopentyloxy-4-methoxybenzamide
- BT: N-(2,6-dichloro-4-hydroxymethylphenyl)-3-cyclopentyloxy-4-methoxybenzamide
- BU: N-(3,5-dichloropyrid-4-yl)-3-dodecyloxy-4-methoxybenzamide
- BV: (R)-N-(3,5-dichloropyrid-4-yl)-3-(exo-8,9,10-trinorbornyl-2-oxy)-4-methoxybenzamide
- BW: (S)-N-(3,5-dichloropyrid-4-yl)-3-(exo-8,9,10-trinorbornyl-2-oxy)-4-methoxybenzamide
- BX: N-(2,6-dichloro-4-nitrophenyl)-3-cyclopentyloxy-4-methoxybenzamide

The letters A to BX are allocated to compounds for easy reference in this specification.

In J. Med. Chem., 16 (4) 332-336 (1973), I.W. Mathieson et al. disclose that a phenyl compound of formula : 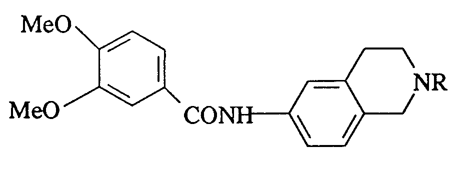
is a hypotensive agent, but do not disclose or suggest that the compound inhibits cyclic AMP phosphodiesterase. I.W. Mathieson et al. also do not disclose or suggest that the benzamide moiety may be substituted by anything other than methoxy.

Chemical Abstracts, 99 (6) 43556z (1983) pertains to an abstract of Japanese Patent Application Publication No. JP-A-5 869 812 which discloses that a phenyl compound of formula : 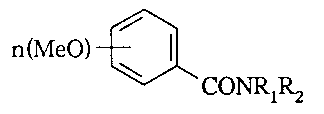
is a hypoglycemic agent, but does not disclose or suggest that the compound inhibits cyclic phosphodiesterase. JP-A-5 869 812 also does not disclose or suggest that the benzamide moiety may be substituted by anything other than methoxy.

Japanese Patent Application Publication No. 62,158,252 (filed 1985, laid open 1987) discloses compounds of the formula : 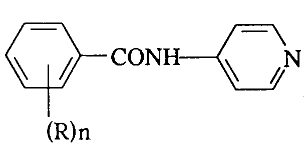
wherein R represents, inter alia, an alkoxy group of 1 to 4 carbon atoms and n represents, inter alia, 2, and reports that they have myocardium contraction enhancing effect. This publication does not disclose or suggest that the pyridyl group may carry any substituents. Neither does it disclose or suggest that the compounds inhibit cyclic AMP phosphodiesterase.

The compounds of general formula (I) and their pharmaceutically acceptable salts exhibit pharmacological activity and accordingly are of use for the preparation of pharmaceutical compositions and in the treatment of humans and other animals. More especially, they are cyclic AMP phosphodiesterase inhibitors, in particular type IV cyclic AMP phosphodiesterase inhibitors, and thus the present invention provides compounds of formula I and their pharmaceutically acceptable salts, and compositions containing compounds of general formula (I) and their pharmaceutically acceptable salts, which are of use in the preparation of a medicine for the treatment of a human or animal patient suffering from, or subject to, conditions which can he ameliorated by the administration of an inhibitor of cyclic AMP
phosphodiesterase. For example, they are useful as bronchodilators and asthma-prophylactic agents and agents for the inhibition of eosinophil accumulation and of the function of eosinophils, e.g. for the treatment of inflammatory airways, disease, especially reversible airway obstruction or asthma, and for the treatment of other diseases and conditions characterised by, or having an aetiology involving, morbid eosinophil accumulation. As further examples of conditions which can be ameliorated by the administration of inhibitors of cyclic AMP phosphodiesterase such as compounds of general formula I there may be mentioned inflammatory diseases, such as atopic dermatitis, urticaria, allergic rhinitis, psoriasis, rheumatic arthritis, ulcerative colitis, Crohn's disease, adult respiratory distress syndrome, and diabetes insipidus, other proliferative skin diseases such as keratosis and various types of dermatitis, conditions associated with cerebral metabolic inhibition, such as cerebral senility, multi-infarct dementia, senile dementia (Alzheimer's disease), and memory impairment associated with Parkinson's disease, and conditions ameliorated by neuroprotectant activity, such as cardiac arrest, stroke, and intermittent claudication.

According to a further feature of the invention there is provided the preparation of a medicine for the treatment of a human or animal patient suffering from, or subject to, conditions which can be ameliorated by the administration of an inhibitor of cyclic AMP phosphodiesterase, for example conditions as hereinbefore described.

Compounds according to formula (I), with the proviso that, when R³ represents an unsubstituted pyrid-4-yl group and Z represents an oxygen atom, R² does not represent an alkyl group containing 2,3 or 4 carbon atoms, constitute an important class of compounds of the invention.

Compounds within the scope of the present invention exhibit positive pharmacological activities as demonstrated by the following tests which are believed to correlate to pharmacological activity in human and other animals.

Compounds of the invention at concentrations from about 10⁻⁹M up to about 10⁻⁵M produced about 50% inhibition of porcine aorta cyclic AMP phosphodiesterase.

Compounds of the invention at concentrations of about 10⁻⁷M to about 10⁻⁴M produced about 50% relaxation of guinea-pig tracheal strips, which had been contracted by treatment with spasmogens such as histamine and carbachol.

Compounds of the invention at intraperitoneal doses from about 1 to about 25 mg/kg or at oral doses from about 1 to about 50 mg/kg inhibited by about 50% the accumulation of eosinophils in the lungs of guinea-pigs.

Compounds of the invention at concentrations from about 10⁻⁸M up to about 10⁻⁵M produced about 50% inhibition of superoxide generation from eosinophils harvested from the peritoneal cavities of guinea-pigs.

Compounds of the invention at oral doses from about 1 to about 50 mg/kg administered 1 hour before challenge inhibited by about 50% ovalbumin- or PAF-induced hyperreactivity in guinea-pigs.

Bronchorelaxant activity was measured in in vivo tests in the anaesthetised guinea-pig according to the method of Dixon and Brodie [J. Physiol., 29, 97-173, (1903)] in which the effects on histamine-induced bronchospasm and mean arterial blood pressure were determined.

Nebulised aerosols generated from aqueous solutions of compounds of the invention were each administered for one minute to the anaesthetised guinea-pigs.

Alternatively, dry powder formulations made up from compounds of the invention and lactose were blown into the airways of the anaesthetised guinea-pigs.

Compounds of the invention produced from about 30% up to about 90% decrease in bronchospasm when administered at effective doses of about 20-80»g, without any significant effect on blood pressure.

Compounds of the invention as oral doses of about 1 to about 50mg/kg, administered one hour before challenge, inhibited by at least 50% ovalbumin-induced eosinophilia in guinea-pigs, which is measured 24 hours after challenge.

Administered at doses of about 1 to about 50mg/kg orally or parenterally or at doses of about 20 to about 500»g intratracheally, compounds of the invention inhibit PAF or ovalbumin-induced microvascular leakage (measured using fluorescein isothiocyanate dextran) by up to 100% in guinea-pigs.

The value of the compounds of the invention is enhanced by their very low mammalian toxicity levels.

Compounds of formula (I) can be prepared by the application or adaptation of known methods, by which is meant methods used heretofore or described in the literature.

Thus, according to a feature of the present invention, compounds of general formula I, wherein R¹, R² and R³ are as hereinbefore defined and Z represents an oxygen atom, are prepared by the reaction of compounds of general Formula II hereinafter depicted, wherein R¹ and R² are as hereinbefore defined and X¹ represents a halogen, e.g. bromine or, preferably, chlorine atom, with compounds of the general formula:-

NH₂R³ III

wherein R³ is as hereinbefore defined, preferably in the presence of a base, for example an alkali metal hydroxide or carbonate, e.g. sodium hydroxide or carbonate, an alkali metal hydride, e.g. sodium hydride, or an amine, preferably a tertiary amine, e.g. triethylamine or pyridine, optionally in an inert solvent, for example dichloromethane, dimethylformamide, or an ether, e.g. diethyl ether or tetrahydrofuran, preferably at a temperature from 0°C to the reflux temperature or at the melting point of the reaction mixture.

According to a further feature of the present invention, compounds of general formula I are prepared by the reaction of compounds of general formula IV, hereinafter depicted, wherein R¹, R³ and Z are as hereinbefore defined, with compounds of the general formula:-

R²X² V

wherein R² is as hereinbefore defined and X² represents a halogen, preferably bromine, atom, preferably in the presence of a base, for example an alkali metal hydride, e.g. sodium hydride, an alkali metal hydroxide or carbonate, e.g. sodium hydroxide or carbonate, or an amine, preferably a tertiary amine, e.g. triethylamine or pyridine, optionally in an inert solvent, for example dichloromethane, dimethylformamide, or an ether, e.g. diethyl ether or tetrahydrofuran, preferably at a temperature from 0°C to the reflux temperature.

According to a further feature of the present invention, compounds of general formula I are prepared by interconversion of other compounds of general formula I.

For example, compounds of general formula I wherein R¹, R² and R³ are as hereinbefore defined and Z represents a sulphur atom are prepared from compounds of general formula I wherein R¹, R² and R³ are as hereinbefore defined and Z represents an oxygen atom, by reaction with phosphorus pentasulphide or 2,4-bis(4-methoxyphenyl)-1,3-dithia-2,4-diphosphetane-2,4-disulphide, preferably in a solvent such as pyridine or toluene, and preferably at a temperature from 0°C to the reflux temperature.

As another example, compounds of general formula I wherein R¹ and R² are as hereinbefore defined, Z represents an oxygen atom and R³ contains an alkylsulphonyl, arylsulphonyl alkylsulphinyl or arylsulphinyl group are prepared by the oxidation of compounds of general formula I wherein R¹ and R² are as hereinbefore defined, Z represents an oxygen atom and R³ contains an alkylthio or arylthio group, preferably by means of reaction with a peroxyacid, e.g. 3-chloro-perbenzoic acid, preferably in an inert solvent, e.g. dichloromethane, preferably at or near room temperature.

As another example, compounds of general formula I wherein R¹, R² and Z are as hereinbefore defined, Z preferably being an oxygen atom, and R³ contains a hydroxymethyl group are prepared by the reduction of compounds of general formula I wherein R¹, R² and Z are as hereinbefore defined and R³ contains an aryloxycarbonyl or, preferably, alkoxycarbonyl group, preferably by means of reaction with an alkali metal borohydride, preferably in an inert solvent, e.g. tetrahydrofuran, preferably at or near room temperature.

As another example, compounds of general formula I wherein R¹, R² and Z are as hereinbefore defined, Z preferably being an oxygen atom, and R³ contains a formyl group are prepared by the oxidation of compounds of general formula I wherein R¹, R² and Z are as hereinbefore defined and R³ contains a hydroxymethyl group, preferably by means of reaction with manganese dioxide, preferably in an inert solvent, e.g. dichloromethane, preferably at or above room temperature, more especially at the reflux temperature.

As another example, compounds of general formula I wherein R¹, R² and Z are as hereinbefore defined, Z preferably being an oxygen atom, and R³ contains an amino group are prepared by the reduction of compounds of general formula I wherein R¹, R² and Z are as hereinbefore defined and R³ contains a nitro group, preferably by means of reaction with iron in acidic conditions, e.g. in acetic acid, preferably at or above room temperature, more especially at the reflux temperature.

As another example, compounds of general formula I wherein R¹, R² and Z are as hereinbefore defined, Z preferably being an oxygen atom, and R³ contains an alkanoylamino or aroylamino group are prepared from compounds of general formula I wherein R¹, R² and Z are as hereinbefore defined and R³ contains an amino group, preferably by means of reaction with the appropriate acid halide or acid anhydride, optionally in an inert solvent, and preferably at a temperature from 0°C to the reflux temperature.

As another example, N-oxides of compounds of general formula I wherein R¹, R² and Z are as hereinbefore defined, Z preferably being an oxygen atom, and R³ represents a heterocyclyl group containing one or more nitrogen ring atoms, are prepared by the oxidation of compounds of general formula I wherein R¹, R² and Z are as hereinbefore defined and R³ represents a heterocyclyl group containing one or more nitrogen ring atoms, preferably by means of reaction with a mixture of hydrogen peroxide and an organic acid, e.g. acetic acid, preferably at or above room temperature at 60-90°C.

By the term "pharmaceutically acceptable salts" as used in this specification is meant salts the counter ions of which are relatively innocuous to the animal organism when used in therapeutic doses so that the beneficial pharmaceutical properties of the parent compounds of general formula I are not vitiated by side-effects ascribable to those counter ions.

Suitable acid addition salts for use in pharmaceuticals may be selected from salts derived from inorganic acids, for example hydrohalides, e.g. hydrochlorides and hydrobromides, phosphates, sulphates and nitrates, and organic acids, for example oxalates, lactates, tartrates, acetates, salicylates, citrates, propionates, succinates, fumarates, maleates, methylene-bis-β-hydroxynaphthoates, gentisates, mesylates, isethionates and di-p-toluoyltartrates.

As will be self-evident to those skilled in the art, some of the compounds of general formula I do not form stable salts. However, acid addition salts are most likely to be formed by compounds of formula I wherein
R³ represents a nitrogen-containing heterocyclyl group and/or wherein R³ contains an amino group as a substituent.

According to a further feature of the invention, acid addition salts of compounds of formula I are prepared by reaction of the parent compounds of formula I with the appropriate acid, by the application or adaptation of known methods.

Alkali and alkaline earth metal salts are also suitable for use in pharmaceuticals, especially sodium salts.

According to a further feature alkali and alkaline earth metal salts are prepared by reaction of the parent compounds of formula I with the appropriate base, by the application or adaptation of known methods. For example, sodium salts can conveniently be prepared by reaction with sodium hydride.

As well as being useful in themselves as active compounds, salts of compounds of formula I are useful for the purposes of purification of the parent compounds of formula I, for example by exploitation of the solubility differences between the salts and the parent compounds, by techniques well known to those skilled in the art.

The parent compounds of formula I can be regenerated from their salts by the application or adaptation of known methods.

For example, parent compounds of general formula I can be regenerated from their acid addition salts by treatment with an alkali, e.g. aqueous sodium bicarbonate solution or aqueous ammonia solution.

Similarly, parent compounds of general formula I can be regenerated from their alkali and alkaline earth metal salts by treatment with an acid, e.g. hydrochloric acid.

In this specification reference to compounds of formula I is intended to include reference to their pharmaceutically acceptable salts, where the context so permits.

It will be apparent to those skilled in the art that certain compounds of general formula I can exhibit isomerism, for example optical isomerism. All isomers within general formula I, and their mixtures, are within the scope of the invention.

Such isomers can be separated from their mixtures, by the application or adaptation of known methods, for example chromatographic techniques, or they may be separately prepared from the appropriate isomers of their intermediates, for example by the application or adaptation of methods described herein.

The starting materials and intermediates can be prepared by the application or adaptation of known methods, for example methods as described in the Reference Examples or their obvious chemical equivalents.

For example, compounds of formula II can be prepared from compounds of general formula Va, hereinafter depicted, wherein R¹ and R² are as hereinbefore defined, by the application or adaptation of known methods for the preparation of acid halides from carboxylic acids. For example, when X¹ represents a chlorine atom, the reaction can be carried out by means of thionyl chloride.

Compounds of formula Va can be prepared by the oxidation of compounds of general formula VI, hereinafter depicted, wherein R¹ and R² are as hereinbefore defined, e.g. by means of reaction with potassium permanganate, or with a mixture of sulphamic acid and sodium chlorite in acetic acid.

Compounds of formula VI can be prepared from compounds of general formula VII, hereinafter depicted, wherein R¹ is as hereinbefore defined, by reaction with compounds of formula V as hereinbefore defined, or alternatively by reaction with compounds of the general formula:-

R²OH VIII

wherein R² is as hereinbefore defined, preferably in the presence of a compound such as diisopropyl azodicarboxylate. 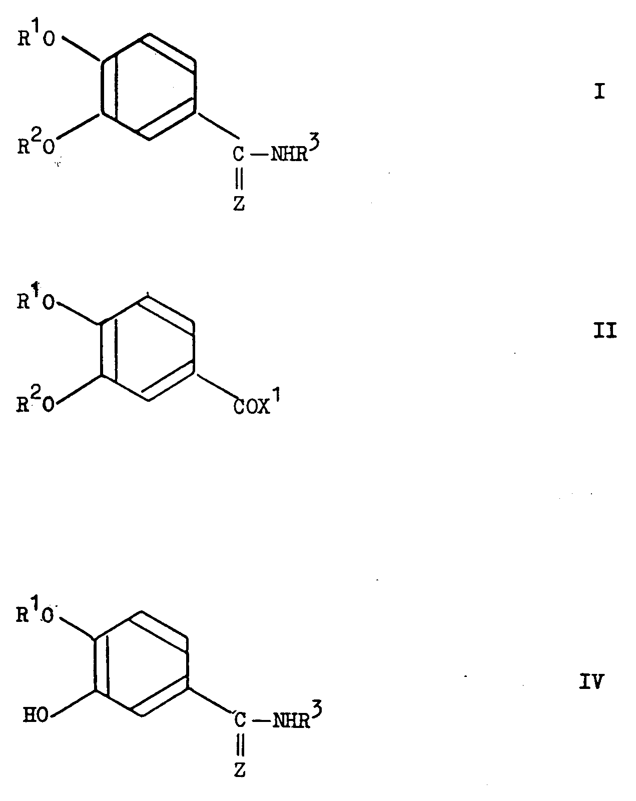 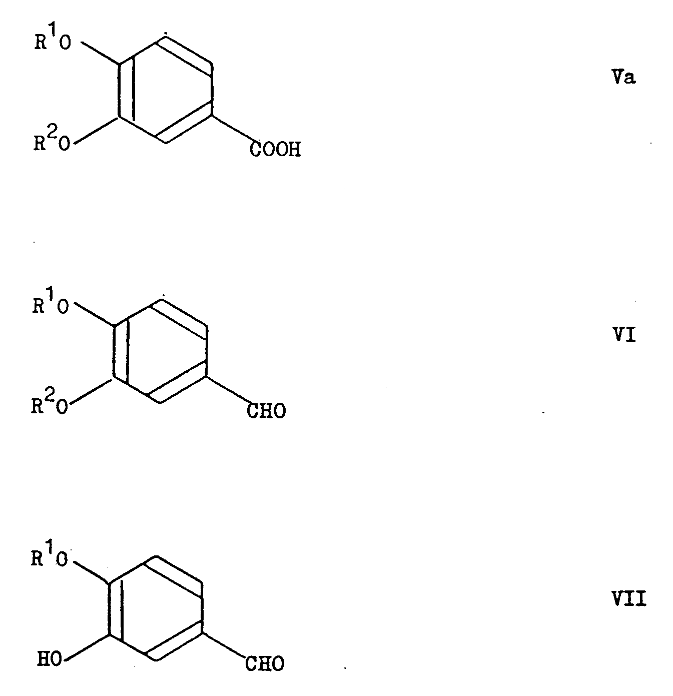
The following Examples illustrate the preparation of the compounds according to the invention and the Reference Examples illustrate the preparation of the intermediates.

In the nuclear magnetic resonance spectra (NMR) the chemical shifts are expressed in ppm relative to tetramethylsilane. Abbreviations have the following significances:-
s = singlet; d = doublet; t = triplet; q = quartet;
m = multiplet; dd = doublet of doublets; b = broad.

### EXAMPLE 1

### Compounds A,B,C,D,E,F,G,H,I,J K,L,M,N,O,P,Q,R,S,T,U,V,W,X,Y and Z

A stirred solution of 2,6-difluoroaniline (1.52g) and triethylamine (1.19g) in dichloromethane (50ml) at room temperature was treated with a solution of 3-cyclopentyloxy-4-methoxybenzoyl chloride (3.0g, prepared as described hereinafter in Reference Example 3) in dichloromethane (50ml), dropwise. The solution was stirred and heated at reflux for 4 hours, then it was cooled, washed with water and dried over magnesium sulphate. The solution was concentrated and the resulting residue was recrystallised from ethyl acetate, to give N-(2,6-difluorophenyl)-3-cyclopentyloxy-4-methoxybenzamide (1.9g), m.p. 158-160°C [NMR(CDCl₃):-1.55-1.7(m,3H),1.8-2.05(m,5H),3.93(s,3H),4.85(m,1H), 6.9(d,1H),6.95-7.03(m,2H),7.2-7.3(m,1H),7.35(bs,1H), 7.45(q,1H),7.53(d,1H); Elemental analysis:- C,65.1; H,5.6;F,10.4;N,4.2%; Calculated:- C,65.7;H,5.5;F,10.9; N,4.0%].

By proceeding in a similar manner, but replacing the 2,6-difluoroaniline by the appropriate quantities of the corresponding aniline derivatives, there were prepared:-
N-(2-chloro-6-fluorophenyl)-3-cyclopentyloxy-4-methoxybenzamide, m.p. 140-142°C [Elemental analysis:-C,62.3; H,5.2;Cl,9.7;N,3.6%; Calculated:- C,62.7;H,5.3;Cl,9.75; N,3.85%];
N-(2-trifluoromethylphenyl)-3-cyclopentyloxy-4-methoxybenzamide, m.p. 127-129°C [Elemental analysis:- C,63.4; H,5.5;F,13.3;N,3.3%; Calculated:- C,63.3;H,5.3;F,15.0; N,3.7%];
N-(2,4,6-trichlorophenyl)-3-cyclopentyloxy-4-methoxybenzamide, m.p. 173°C [Elemental analysis:- C,55.2; H,4.4;Cl,26.4;N,3.1; Calculated:- C,55.0;H,4.4;Cl,25.6; N,3.4%];
N-(2,6-dibromophenyl)-3-cyclopentyloxy-4-methoxybenzamide, m.p. 133°C [Elemental analysis:- C,48.5; H,4.0;Br,33.9;N,2.85%; Calculated:- C,48.6;H,4.1; Br,34.1;N,3.0%];
N-(2-chloro-6-methylphenyl)-3-cyclopentyloxy-4-methoxybenzamide, m.p. 138-140°C [Elemental analysis:-C,66.3;H,6.2;Cl,10.3;N,3.8%; Calculated:- C,66.75; H,6.2;Cl,9.85;N,3.9%];
N-(2,6-dichlorophenyl)-3-cyclopentyloxy-4-methoxybenzamide, m.p. 138-140°C [Elemental analysis:- C,59.8; H,5.1;Cl,19.1;N,3.3%; Calculated:- C,60.0;H,5.0;Cl,18.65; N,3.7%];
N-(2-fluorophenyl)-3-cyclopentyloxy-4-methoxybenzamide, m.p. 137°C [Elemental analysis:- C,69.3;H,6.2;F,5.7; N,4.0%; Calculated:- C,69.3;H,6.1;F,5.8;N,4.25%];
N-phenyl-3-cyclopentyloxy-4-methoxybenzamide, m.p. 169-173°C [Elemental analysis:- C,73.2;H,6.7; N,4.2%; Calculated:- C,73.3;H,6.8;N,4.5%];
N-(2-methoxyphenyl)-3-cyclopentyloxy-4-methoxybenzamide, m.p. 132-134°C [Elemental analysis:- C,70.1; H,6.8;N,4.0%; Calculated:- C,70.4;H,6.8;N,4.1%];
N-(2-chlorophenyl)-3-cyclopentyloxy-4-methoxybenzamide, m.p. 122-124°C [Elemental analysis:- C,65.8;H,5.8;Cl,10.5; N,3.9%; Calculated:- C,66.0;H,5.8;Cl,10.25;N,4.05%];
N-(3-chlorophenyl)-3-cyclopentyloxy-4-methoxybenzamide, m.p. 110-112°C [Elemental analysis:- C,65.9;H,6.5;Cl,9.8; N,3.7%; Calculated:- C,66.0;H,5.8;Cl,10.25;N,4.05%];
N-(4-methoxyphenyl)-3-cyclopentyloxy-4-methoxybenzamide, m.p. 182-184°C [Elemental analysis:- C,68.7; H,6.6;N.3.8%;Calculated for C₂₀H₂₃NO₄:½H₂O:- C,68.55; H,6.9;N.4.0%];
N-(2,6-dimethylphenyl)-3-cyclopentyloxy-4-methoxybenzamide, m.p. 130-131°C [Elemental analysis:- C,74.2; H,7.4;N,4.1%; Calculated:- C,74.3;H,7.4;N,4.13%];
N-(2-methylthiophenyl)-3-cyclopentyloxy-4-methoxybenzamide, m.p. 128-130°C [Elemental analysis:- C,67.6; H,6.5;N,3.9;S,8.9%; Calculated:- C,67.2;H,6.5;N,3.9; S,9.0%];
N-(2-bromophenyl)-3-cyclopentyloxy-4-methoxybenzamide, m.p. 126-128°C [Elemental analysis:- C,58.2;H,5.1; Br,20.4;N,3.5%; Calculated:- C,58.5;H,5.2;Br,20.5; N,3.6%];
N-(2-methoxycarbonylphenyl)-3-cyclopentyloxy-4-methoxybenzamide, m.p. 105-107°C [Elemental analysis:- 68.4; H,6.35;N,3.7%; Calculated:- 68.3;6.3;N,3.8%];
N-(2-aminosulphonylphenyl)-3-cyclopentyloxy-4-methoxybenzamide, m.p. 248°C [Elemental analysis:- C,58.0; H,5.5;N,6.9%; Calculated:- C,58.45;H,5.7;N,7.2%];
N-(2-benzoylphenyl)-3-cyclopentyloxy-4-methoxybenzamide, m.p. 106-107°C [Elemental analysis:- C,75.5;H,6.3; N,3.3%; Calculated:- C,75.2;H,6.1;N,3.4%];
N-(2-cyanophenyl)-3-cyclopentyloxy-4-methoxybenzamide, m.p. 170-172°C [Elemental analysis:- C,71.0;H,6.0; N,8.1%; Calculated:- C,75.2;H,6.1;N,3.4%];
N-(2,5-dichlorophenyl)-3-cyclopentyloxy-4-methoxybenzamide, m.p. 117-119°C [Elemental analysis:- C,59.7; H,5.0;Cl,18.5;N,3.7%; Calculated:- C,60.0;H,5.0; Cl,18.65;N,3.7%];
N-(3-methylphenyl)-3-cyclopentyloxy-4-methoxybenzamide, m.p. 147-149°C [Elemental analysis:- C,73.8; H,7.1;N,4.2%; Calculated:- C,73.8;H,7.1;N,4.3%];
N-(2-nitrophenyl)-3-cyclopentyloxy-4-methoxybenzamide, m.p. 130-132°C [Elemental analysis:- C,64.0;H,5.7; N,7.4%; Calculated:- C,64.0;H,5.7;N,7.9%];
N-(2-dimethylaminophenyl)-3-cyclopentyloxy-4-methoxybenzamide,in the form of a brown oil [Elemental analysis:- C,71.5;H,7.4;N,7.4%; Calculated:- C,71.2; H,7.4;N,7.9%];
N-(2-acetylphenyl)-3-cyclopentyloxy-4-methoxybenzamide, m.p. 126-127°C [Elemental analysis:- C,71.0;H,6.6; N,3.9%; Calculated:- C,71.4;H,6.6;N,4.0%]; and
N-(2-hydroxyphenyl)-3-cyclopentyloxy-4-methoxybenzamide, m.p. 169-171°C [Elemental analysis:- C,69.5; H,6.5;N,3.9%; Calculated:- C,69.7;H,6.5;N,4.3%].

### EXAMPLE 2

### Compound AA

A stirred solution of N-(2-methylthiophenyl)-3-cyclopentyloxy-4-methoxybenzamide (1.80g; prepared as described hereinbefore in Example 1) was treated with a solution of 3-chloroperbenzoic acid (3.60g; 85% pure) in dichloromethane (72ml), dropwise, and then it was stirred at room temperature for 5 hours. The reaction mixture was washed with saturated aqueous sodium bicarbonate solution and then with water, and then it was dried over magnesium sulphate. Concentration gave N-(2-methylsulphonylphenyl)-3-cyclopentyloxy-4-methoxybenzamide, (1.12g), in the form of a white solid, m.p. 119-121°C [NMR(CDCl₃):- 1.52-2.16 (m,8H),3.1(s,3H),3.94(s,3H),4.9(m,1H),6.96(d,1H),7.46 (m,1H),7.6(m,2H),7.7(t,1H),7.95(d,1H),8.68(d,1H);
Elemental analysis:- C,61.6;H,6.0;N,3.5;S,8.5%; Calculated:- C,61.7;H,5.95;N,3.6;S,8.5%].

### EXAMPLE 3

### Coumpounds AB, AC and AD

By proceeding in a manner similar to that described hereinbefore in Example 1, but using the appropriate quantities of the corresponding acid chlorides, prepared as described hereinafter in Reference Example 3, there were prepared:-
N-(2,6-difluorophenyl)-3-cyclohexyloxy-4-methoxybenzamide, m.p. 60°C [Elemental analysis:- C,66.1; H,6.3;N,3.3%; Calculated:- C,66.5;H,5.9;N,3.9%]; N-(2,6-difluorophenyl)-3-butoxy-4-methoxybenzamide, m.p. 150-152°C. [Elemental analysis:-C,64.6;H,5.8;N,4.2; Calculated:- C,64.5;H,5.7; N,4.2%]; and
N-(2,6-difluorophenyl)-3-propoxy-4-methoxybenzamide, m.p. 170-174°C. [Elemental analysis:- C,63.4;H,5.4; N,4.4%; Calculated:- C,63.5;H,5.3;N,4.4%].

### EXAMPLE 4

### Compound AE

3-Cyclopentyloxy-4-methoxybenzoyl chloride (13.3g) and 2-chloroaniLine (6.6g) were dissolved in pyridine (50ml) and the solution was allowed to stand at room temperature for 1 hour. Phosphorus pentasulphide (13g) was added and the stirred mixture was heated at 110°C for 1.5 hours. After cooling to room temperature the mixture was poured into an ice-cold solution of concentrated hydrochloric acid (100ml) in water (400ml). The mixture was stirred for 1 hour and the yellow solid was collected, washed with water and subjected to flash chromatography on silica gel, eluting with a mixture of cyclohexane and ethyl acetate (3:1v/v), to give N-(2-chlorophenyl)-3-cyclopentyloxy-4-methoxy(thiobenzamide) (5.4g), m.p. 129-131°C [Elemental analysis:- C,62.6;H,5.5;N,3.9; S,8.9%; Calculated:- C,63.1;H,5.6;N,3.9;S,8.9%].

### EXAMPLE 5

### Compounds AF, AG, AH, AI, AJ, AK AL, AM and AN

A stirred solution of 4-chloropyrid-3-ylamine (1.94g) and 3-cyclopentyloxy-4-methoxybenzoyl chloride (3.85g) in pyridine (50ml) was heated at 80°C for 7 hours and then it was allowed to stand overnight. The reaction mixture was evaporated, to give a brown oil, which was subjected to mplc on silica gel, using diethyl ether as eluent, to give N-(4-chloropyrid-3-yl)-3-cyclopentyloxy-4-methoxybenzamide (3.1g), m.p. 130-132°C.

By proceeding in a similar manner, but using the appropriate quantities of the appropriate amines instead of the 4-chloropyrid-3-ylamine used as a starting material, there were prepared:-
N-pyrid-2-yl-3-cyclopentyloxy-4-methoxybenzamide, m.p. 92-94°C;
N-pyrazin-2-yl-3-cyclopentyloxy-4-methoxybenzamide, m.p. 80-82°C;
N-pyrimidin-2-yl-3-cyclopentyloxy-4-methoxybenzamide, m.p. 108-110°C;
N-(3-methylpyrid-2-yl)-3-cyclopentyloxy-4-methoxybenzamide, m.p. 55°C;
N-pyrid-3-yl-3-cyclopentyloxy-4-methoxybenzamide, m.p. 170-172°C;
N-(3-chloropyrid-2-yl)-3-cyclopentyloxy-4-methoxybenzamide, m.p. 138-140°C;
N-(3-chloropyrid-4-yl)-3-cyclopentyloxy-4-methoxybenzamide, m.p. 124-126°C
N-pyrid-4-yl-3-cyclopentyloxy-4-methoxybenzamide, m.p. 163-165°C.

### EXAMPLE 6

### Compound AO

4-Amino-3,5-dichloropyridine (4.0g) and 3-cyclopentyloxy-4-methoxybenzoyl chloride (6.26g) were intimately ground together in a mortar with a pestle, and transferred to a round-bottomed flask. The mixture was melted, using a hot air gun external to the flask, stirring with a magnetical stirrer. After 10 minutes, heating was ceased and the melt was allowed to cool. The resulting material was triturated with dichloromethane and the residual solid was filtered off. The filtrate was concentrated to give a fawn solid, which was subjected to flash chromatography on silica gel, eluting with diethyl ether, to give N-(3,5-dichloropyrid-4-yl)-3-cyclopentyloxy-4-methoxybenzamide (1.87g), m.p. 155-157°C.
[Elemental analysis: C,56.3;H,4.7;N,7.2;Cl,18.4%; calculated: C,56.7;H,4.76;N,7.35;Cl,18.6%; IR spectrum:- 1661cm⁻¹, 3244cm ^{-1]}.

### EXAMPLE 7

### Compound AP

By proceeding in a manner similar to that described hereinbefore in Example 1, but replacing the 2,6-difluoroaniline used as a starting material by the appropriate quantity of 4-amino-3,5-dimethylisoxazole, there was prepared N-(3,5-dimethylisoxazol-4-yl)-3-cyclopentyloxy-4-methoxybenzamide, m.p. 150-152°C.
[Elemental analysis: C,65.6;H,6.8;N,8.5%; calculated: C,65.4;H,6.7;N,8.5%].

### EXAMPLE 8

### Compounds AO,AY,BC,BG,BL,BQ,BS,BX,AX,AZ,AW,BV and BW

A suspension of sodium hydride (60% dispersion in oil; 2.2g) in dry tetrahydrofuran (25ml) at 15-20°C was treated portionwise with a solution of 4-amino-3,5-dichloropyridine (4.5g; prepared as described in Reference Example 5) in dry tetrahydrofuran (40ml), with cooling. The mixture was stirred for a further 30 minutes, and then it was cooled to 10°C and treated with a solution of 3-cyclopentyloxy-4-methoxybenzoyl chloride (6.4g) in dry tetrahydrofuran (40ml), dropwise, during 45 minutes at 10°C. The mixture was stirred at 10°C for 30 minutes and was then treated with dilute hydrochloric acid (50ml;1N), followed by dichloromethane (75ml). The layers were separated and the aqueous layer was washed with a further quantity of dichloromethane (25ml). The combined organic layers were washed with water (50ml), with saturated aqueous sodium bicarbonate solution (100ml), and with water (50ml), dried over magnesium sulphate and evaporated to dryness. The resulting residue was recrystallised from isopropanol, to give N-(3,5-dichloropyrid-4-yl)-3-cyclopentyloxy-4-methoxybenzamide (7.0g).

By proceeding in a similar manner, but replacing the 4-amino-3,5-dichloropyridine used as a starting material by the appropriate quantities of the appropriate amines, and optionally using dimethylformamide instead of tetrahydrofuran, there were prepared:-
N-(3,5-dibromopyrid-4-yl)-3-cyclopentyloxy-4-methoxybenzamide, m.p. 160-162°C [Elemental analysis:-C,46.4;H,3.9;N,6.1%; calculated:- C,46.0;H,3.9;N,6.0%];
N-(3,5-dimethylpyrid-4-yl)-3-cyclopentyloxy-4-methoxybenzamide, m.p. 77-80°C [Elemental analysis:-C,67.2;H,6.9;N,7.8%; calculated:- C,67.0;H,7.3;N,7.8%];
N-(2,6-dichloro-4-cyanophenyl)-3-cyclopentyloxy-4-methoxybenzamide, m.p. 170-172°C [Elemental analysis:-C,59.1;H,4.5;N,7.0;Cl,17.5%; calculated:- C,59.3;H,4.5; N,6.9;Cl,17.5];
N-(2,6-dichloro-4-methoxycarbonylphenyl)-3-cyclopentyloxy-4-methoxybenzamide, m.p. 158-160°C [Elemental analysis:- C,57.4;H,4.9;N,3.2;Cl,16.4%; calculated:-C,57.5;H,4.8;N,3.2;Cl,16.2%];
N-(2,3,5-trifluoropyrid-4-yl)-3-cyclopentyloxy-4-methoxybenzamide, m.p. 144-146°C [Elemental analysis:C,59.3;H,4.9;N,7.5%; calculated:- C,59.0;H,4.7; N,7.65%];
N-(2,6-dichloro-4-ethoxycarbonylphenyl)-3-cyclopentyloxy-4-methoxybenzamide, m.p. 164-166°C;
N-(2,6-dichloro-4-nitrophenyl)-3-cyclopentyloxy-4-methoxybenzamide, m.p. 154-156°C;
N-(3,5-dichloropyrid-4-yl)-3-cyclohexyloxy-4-methoxybenzamide, m.p. 170°C [Elemental analysis:- C,57.8; H,5.1;N,7.0;Cl,17.8%; calculated:- C,57.7;H,5.1;N,7.1; Cl,17.9%];
N-(3,5-dichloropyrid-4-yl)-3-butoxy-4-methoxybenzamide, m.p. 165-167°C [Elemental analysis:- C,55.1;H,4.8; N,7.6;Cl,19.2%; calculated:- C,55.3;H,4.9;N,7.6; Cl,19.2%];
N-(3,5-dichloropyrid-4-yl)-3-(exo-8,9,10-trinorbornyl-2-oxy)-4-methoxybenzamide, m.p. 149-150°C [Elemental analysis:- C,58.8;H,4.9;N,6.7%; calculated:- C,59.0; H,5.0;N,6.9%];
(R)-N-(3,5-dichloropyrid-4-yl)-3-(exo-8,9,10-trinorbornyl-2-oxy)-4-methoxybenzamide, m.p. 155-156°C [Elemental analysis:- C,58.8;H,5.0;N,6.8%]; and
(S)-N-(3,5-dichloropyrid-4-yl)-3-(exo-8,9,10-trinorbornyl-2-oxy)-4-methoxybenzamide, m.p. 156-157°C.

### EXAMPLE 9

### Compound AV

A stirred suspension of N-(3,5-dichloropyrid-4-yl)-3-cyclopentyloxy-4-methoxybenzamide (2.0g; prepared as described in Example 6) in glacial acetic acid (8ml) was treated with an aqueous solution of hydrogen peroxide (6ml;27.5%). The mixture was stirred for 3 hours at 70-80°C and then it was treated with a further portion of hydrogen peroxide solution (4ml), and the solution was stirred for a further 12 hours. The solution was then cooled, basified by treatment with concentrated aqueous sodium hydroxide solution, and extracted with dichloromethane (2x30ml). The organic extract was washed with brine (30ml), dried over magnesium sulphate and evaporated. The resulting residue was recrystallised from ethyl acetate, to give 3,5-dichloro-4-(3-cyclopentyloxy-4-methoxybenzamido)-pyridine-N-oxide (0.73g), m.p. 118-120°C [Elemental analysis:-C,53.0;H,4.4;N,6.8%; calculated for C₁₈H₁₈O₄N₂Cl₂:0.5H₂O:- C,53.2;H,4.7;N,6.9%].

### EXAMPLE 10

### Compound BE

A stirred solution of N-(3,5-dichloropyrid-4-yl)-3-cyclopentyloxy-4-methoxybenzamide (2.0g; prepared as described in Example 6) in toluene (50ml) was treated with 2,4-bis(4-methoxyphenyl)-1,3-dithia-2,4-diphosphetane-2,4-disulphide (3.0g), and the mixture was heated at 100°C for 2 hours. After cooling to room temperature and filtration, the filtrate was concentrated in vacuo, to give a yellow oil. This oil was subjected to flash chromatography on silica gel, using a mixture of pentane and ethyl acetate (8:2v/v) as eluent, to give N-(3,5-dichloropyrid-4-yl)-3-cyclopentyloxy-4-methoxy(thiobenzamide) (0.64g) m.p. 118-119°C [Elemental analysis:- C,54.1;H,4.6;Cl,17.4; N,6.8%; calculated:- C,54.4;H,4.6;Cl,17.85;N,7.05%].

### EXAMPLE 11

### Compound BI

A solution of N-(2,6-dichloro-4-nitrophenyl)-3-cyclopentyloxy-4-methoxybenzamide (1.5g; prepared as described in Example 8) in glacial acetic acid (22ml) was treated with iron pin dust (1.3g) and the mixture was heated with stirring at 90°C for 1 hour. The reaction mixture was cooled, basified to pH8 by treatment with saturated aqueous sodium carbonate solution, and extracted with ethyl acetate (2 x 150ml). The combined organic extract was dried over magnesium sulphate and concentrated in vacuo, to give a white solid. This solid was subjected to flash chromatography, eluting with a mixture of ethyl acetate and pentane (1:1v/v), to give N-(2,6-dichloro-4-aminophenyl)-3-cyclopentyloxy-4-methoxybenzamide (0.8g), m.p. 170-172°C [Elemental analysis:- C,54.8;H,5.04; N,6.5;Cl,17.4%; calculated:- C,57.7;H,5.1;N,7.1; Cl,17.9%].

### EXAMPLE 12

### Compound BM

Acetic anhydride (10ml) was treated with N-(2,6-dichloro-4-aninophenyl)-3-cyclopentyloxy-4-methoxybenzamide (0.8g; prepared as described in Example 11), and the reaction mixture was stirred for 2 hours and left to stand overnight. It was then poured into water (100ml), and extracted with ethyl acetate (100ml) and then with dichloromethane (100ml). The organic extracts were combined, dried over magnesium sulphate, and evaporated, to give N-(4-acetylamino-2,6-dichlorophenyl)-3-cyclopentyloxy-4-methoxybenzamide (0.4g), m.p. 250-252°C [Elemental analysis:- C,57.6;H,5.05;N,6.3;Cl,16.1%; calculated:C,57.5;H,5.1;N,6.4;Cl,16.2%].

### EXAMPLE 13

### Compounds BN and BU

A stirred solution of N-(3,5-dichloropyrid-4-yl)-3-hydroxy-4-methoxybenzamide (2.0g; prepared as described in Reference Example 12) in dimethylformamide (20ml) at room temperature under nitrogen was treated portionwise with a suspension of sodium hydride (60% dispersion in oil; 0.26g), and then it was stirred for a further hour at room temperature. It was then treated dropwise with 1-bromononane (1.2ml) and stirred at 60°C for 5 hours. The solution was then cooled to room temperature , diluted with water (60ml), and extracted with ethyl acetate (2x100ml). The combined organic extracts were dried over magnesium sulphate and evaporated, to give a white solid, which was subjected to flash chromatography on silica gel, eluting with t-butyl methyl ether, to give N-(3,5-dichloropyrid-4-yl)-3-nonyloxy-4-methoxybenzamide (0.56g), m.p. 151-153°C [Elemental analysis:- C,60.3;H,6.45;N,6.3%; calculated:- C,60.1;H,6.4;N,6.4%].

By proceeding in a similar manner, but using the appropriate quantity of 1-bromododecane, there was prepared N-(3,5-dichloropyrid-4-yl)-3-dodecyloxy-4-methoxybenzamide, m.p.143-145°C.

### EXAMPLE 14

### Compound BO

A solution of N-(2,6-dichloro-4-hydroxymethylphenyl)-3-cyclopentyloxy-4-methoxybenzamide (4.4g) in dichloromethane (30ml) was treated with activated manganese dioxide (6.2g), and the mixture was stirred at reflux for 24 hours. The mixture was filtered, the filtrate was evaporated, and the resulting residue was subjected to flash chromatography on silica gel, eluting with ethyl acetate, to give N-(2,6-dichloro-4-formylphenyl)-3-cyclopentyloxy-4-methoxybenzamide (2.4g), m.p. 96-98°C [Elemental analysis:- C,59.0; H,5.1;N,3.1%; calculated:- C,58.8;H,4.7;N,3.4%].

### EXAMPLE 15

### Compound BT

A stirred solution of N-(2,6-dichloro-4-ethoxycarbonylphenyl)-3-cyclopentyloxy-4-methoxybenzamide (6.1g;prepared as described in Example 8) in dry tetrahydrofuran (80ml) at room temperature under argon was treated dropwise with a solution of lithium borohydride in tetrahydrofuran (115ml;2M). The mixture was stirred overnight and then it was treated portionwise with saturated brine (200ml) and stirred for 30 minutes. The organic layer was then washed with water, dried over magnesium sulphate and evaporated. The resulting residue was subjected to flash chromatography on silica gel, to give N-(2,6-dichloro-4-hydroxymethylphenyl)-3-cyclopentyloxy-4-methoxybenzamide (4.4g), m.p. 174-176°C [Elemental analysis:- C,57.1;H,5.4;N,2.9%; calculated for C₂₀H₂₁O₄NCl₂:0.5H₂O:- C,57.3;H,5.3;N,3.3%].

### EXAMPLE 16

### Compound BR

A solution of N-(3,5-dichloropyrid-4-yl)-3-cyclopentyloxy-4-methoxybenzamide (3.8g; prepared as described in Example 6) in dry tetrahydrofuran (25ml) was treated with a suspension of sodium hydride (60% dispersion in oil; 0.40g), and the mixture was stirred until effervescence had ceased and a solution had formed. This solution was evaporated in vacuo and the resulting residue was triturated with t-butyl methyl ether (20ml). The resulting off-white solid was filtered off, quickly washed with t-butyl methyl ether (2x20ml) and dried, to give the sodium salt of N-(3,5-dichloropyrid-4-yl)-3-cyclopentyloxy-4-methoxybenzamide (3.5g), m.p. 265-270°C (with decomposition) [NMR(DMSO-D₆):- 1.52-1.93(m,8H),4.77(s,3H),4.75-4.80 (m,1H),6.98(d,1H),7.58(dd,1H),7.60(s,1H),8.20(s,2H); IR spectrum:- strong peak at 1508cm⁻¹, with no peaks at or near 1661cm⁻¹ nor 3244cm⁻¹, which would have been characteristics of the starting material].

### EXAMPLE 17

### Compounds AU, BF and BP

By proceeding in a manner similar to that described in Example 5, but replacing the 4-chloropyrid-3-ylamine used as a starting material by the appropriate quantities of the corresponding aniline derivatives, there were prepared:-
N-(2,4,6-trifluorophenyl)-3-cyclopentyloxy-4-methoxybenzamide, m.p. 160-162°C [Elemental analysis:- C,62.5; H,5.0;N,3.6%; calculated:- C,62.5;H,5.0;N,3.8%]; and N-(2,6-dichloro-4-methoxyphenyl)-3-cyclopentyloxy-4-methoxybenzamide, m.p. 126-128°C [Elemental analysis:C,57.9;H,4.9;N,3.2%; calculated:- C,58.5;H,5.2;N,3.4%].

By again proceeding in a similar manner, but replacing the 4-chloropyrid-3-ylamine and the 3-cyclopentyloxy-4-methoxybenzoyl chloride by the appropriate quantities of 2,6-dichloroaniline and 3-(exo-8,9,10-trinorbornyl-2-oxy)-4-methoxybenzoyl chloride (prepared as described in Reference Example 14), there was prepared N-(2,6-dichlorophenyl)-3-(exo-8,9,10-trinorbornyl-2-oxy)-4-methoxybenzamide, m.p. 106-107°C [Elemental analysis:- C,61.8;H,5.2;N,3.2%; calculated:- C,62.1;H,5.2;N,3.45%].

### EXAMPLE 18

### Compounds AQ, AS, AT, BD, BH, BJ and BK

By proceeding in a manner similar to that described in Example 6, but replacing the 4-amino-3,5-dichloropyridine used as a starting material by the appropriate quantities of the corresponding amines, there were prepared: N-(4,6-dichloropyrimid-5-yl)-3-cyclopentyloxy-4-methoxybenzamide, m.p. 191-193°C [Elemental analysis:-C,53.1;H,4.4;Cl,18.6;N,10.9%; calculated:- C,53.1; H,4.5;Cl,18.6;N,10.8%];
N-(2,3,5,6-tetrafluoropyrid-4-yl)-3-cyclopentyloxy-4-methoxybenzamide, m.p. 178-180°C [Elemental analysis:-C,56.0;H,4.1;N,7.2%; calculated:-
C,56.25;H,4.2;N,7.3%];
N-(3,5-dichloro-2,6-difluoropyrid-4-yl)-3-cyclopentyloxy-4-methoxybenzamide, m.p. 188-190°C [Elemental analysis:- C,51.5;H,3.8;N,6.8;Cl,17.0%; calculated:-C,51.8;H,3.9;N,6.7;Cl,17.0%];
N-(5-cyano-3-methylisothiazol-4-yl)-3-cyclopentyloxy-4-methoxybenzamide, m.p. 163-164°C [Elemental analysis:- C,60.0;H,5.3;N,11.7%; calculated:- C,60.5; H,5.85;N,11.8%];
N-(2,6-dichloro-4-carbamoylphenyl)-3-cyclopentyloxy-4-methoxybenzamide, m.p. 245-247° [Elemental analysis:-C,54.0;H,4.5;N,6.4%; calculated:- C,54.4;H,5.0; N,6.35%]; and
N-(3-chloro-2,5,6-trifluoropyrid-4-yl)-3-cyclopentyloxy-4-methoxybenzamide, m.p. 188-190°C [Elemental analysis:- C,53.7;H,3.95;N,6.81;Cl,8.9%; calculated:-C,53.94;H,4.0;N,7.0;Cl,8.85%].

By again proceeding in a similar manner, but replacing the 4-amino-3,5-dichloropyridine and the 3-cyclopentyloxy-4-methoxybenzoyl chloride by the appropriate quantities of 4-amino-3,5-dibromopyridine and 3-butoxy-4-methoxybenzoyl chloride (prepared as described in Reference Example 3), there was prepared N-(3,5-dibromopyrid-4-yl)-3-butoxy-4-methoxybenzamide, m.p. 160-162°C [Elemental analysis:- C,44.6;H,3.9; N,6.1%; calculated:- C,44.6;H,4.0;N,6.1%].

### EXAMPLE 19

### Compounds AR, BA and BB

By proceeding in a manner similar to that described in Example 1, but replacing the 2,6-difluoro-aniline used as starting material by the appropriate quantities of the corresponding amines, there were prepared:-
N-(4-nitrophenyl)-3-cyclopentyloxy-4-methoxybenzamide, m.p.178-180°C [Elemental analysis:- C,64.1;H,5.7; N,7.5%; calculated:- C,64.0;H,5.7;N,7.9%];
N-(3-methyl-5-bromoisothiazol-4-yl)-3-cyclopentyloxy-4-methoxybenzamide, m.p. 160-162°C [Elemental analysis:-C,50.0;H,4.7;N,6.8%; calculated:- C,49.6;H,4.7;N,6.8%]; and
N-(3,5-dimethylisothiazol-4-yl)-3-cyclopentyloxy-4-methoxybenzamide, m.p. 140-141°C [Elemental analysis:-C,62.4;H,6.35;N,8.0%; calculated:- C,62.4;H,6.4;N,8.1%].

### REFERENCE EXAMPLE 1

A stirred solution of 3-hydroxy-4-methoxybenzaldehyde (2.00g) in dry dimethylformamide (20ml) was treated portionwise with sodium hydride (60% in oil; 0.56g) and the mixture was then heated for 1 hour at 50°C. It was then treated dropwise with cyclopentyl bromide (2.36g) and was stirred and heated at 50°C for 22 hours. The solution was diluted with water (100ml) and extracted with diethyl ether (2x100ml). The ethereal extracts were combined, dried over magnesium sulphate and concentrated, to give 3-cyclopentyloxy-4-methoxybenzaldehyde (1.65g) in the form of a golden oil.

By proceeding in a similar manner, but using the appropriate quantities of cyclohexyl bromide, butyl bromide and propyl bromide, respectively, there were prepared:-
3-cyclohexyloxy-4-methoxybenzaldehyde in the form of a golden oil [Elemental analysis:- C,71.8;H,7.8%; Calculated:- C,71.8;H,7.7%];
3-butoxy-4-methoxybenzaldehyde in the form of a light brown oil [NMR(CDCl₃):- 1.0(t,3H),1.5(m,2H),1.9(m,2H), 3.96(s,3H),4.1(t,2H),6.96(d,1H),7.4(m,2H),9.8(s,1H)]; and 3-propoxy-4-methoxybenzaldehyde [NMR(CDCl₃):-9.85(s,1H),7.4(dd,1H),7.4(d,1H),7.0(d,1H),4.05(t,2H), 4.0(s,3H),1.9(m,2H),1.06(t,3H)].

### REFERENCE EXAMPLE 2

A stirred saturated aqueous solution of potassium permanganate (100ml) was treated with 3-cyclopentyloxy-4-methoxybenzaldehyde (7.4g; prepared as described hereinbefore in Reference Example 1) and sodium carbonate (3.4g) and the mixture was stirred at 50°C for 1 hour, and then cooled to room temperature. The reaction mixture was acidified by treatment with concentrated hydrochloric acid and then it was treated with aqueous sodium bisulphite solution until a colourless solution was obtained. The reaction mixture was extracted with dichloromethane (2x100ml) and the organic extracts were dried over magnesium sulphate and concentrated. The resulting residue was recrystallised from diethyl ether, to give 3-cyclopentyloxy-4-methoxybenzoic acid (4.78g) in the form of white crystals. [NMR(CDCl₃):- 1.7(s,2H),1.8-2.2 (m,6H),3.95(s,3H),4.85(s,1H),6.9(bs,1H)7.6(bs,1H), 7.8(s,1H),9.8(s,1H); Elemental analysis:- C,65.6; H,6.8%; Calculated:- C,66.1;H,6.8%].

By proceeding in a similar manner, but using the appropriate quantities of the corresponding benzaldehyde derivatives, prepared as described hereinbefore in Reference Example 1, there were prepared:-
3-cyclohexyloxy-4-methoxybenzoic acid in the form of a white solid, m.p. 158-160°C [NMR(CDCl₃):- 1.2-2.1 (m,10H),3.94(s,3H),4.3(m,1H),6.9(d,1H),7.6(s,1H),7.75 (d,1H)];
3-butoxy-4-methoxybenzoic acid in the form of a white solid, m.p. 130-132°C [NMR(CDCl₃):- 1.0 (t,3H), 1.5 (m,2H),1.85(m,2H),3.95(s,3H),4.1(t,2H),6.92(d,2H),7.6 (s,1H),7.75(d,1H)]; and
3-propoxy-4-methoxybenzoic acid ((NMR(CDCl₃):- 7.76 (dd,1H),7.6(d,1H),6.9(d,1H),4.04(t,2H),3.94(s,3H),1.9 (m,2H),1.05(t,3H)].

### REFERENCE EXAMPLE 3

Stirred thionyl chloride (20ml) was treated with 3-cyclopentyloxy-4-methoxybenzoic acid (5.0g; prepared as described hereinbefore in Reference Example 2) portionwise and the solution was then heated at 85°C for 3 hours. Toluene (50ml) was added and the mixture was concentrated to give 3-cyclopentyloxy-4-methoxybenzoyl chloride (4.12g) in the form of an oil which slowly crystallised. [NMR(CDCl₃):- 1.6-1.7 (m,2H),1.8-1.95(m,4H),1.94-2.05(m,2H),3.94(s,3H),4.85 (m,1H),6.9(d,1H),7.55(d,1H),7.8(q,1H); Elemental analysis:- C,61.3;H,5.94;Cl,13.9%; Calculated:- C,61.3; H,5.94;Cl,13.92%].

By proceeding in a similar manner, but using the appropriate quantities of the corresponding benzoic acid derivatives, prepared as described hereinbefore in Reference Example 2, there were prepared:-
3-cyclohexyloxy-4-methoxybenzoyl chloride in the form of a colourless solid;
3-butoxy-4-methoxybenzoyl chloride in the form of a light brown oil; and
3-propoxy-4-methoxybenzoyl chloride [(NMR(CDCl₃):- 7.82 (dd,1H),7.53(d,1H),6.92(d,1H),4.03(t,2H),3.96(s,3H),1.89 (m,2H),1.06(t,3H)].

### REFERENCE EXAMPLE 4

A stirred solution of 4-aminopyridine (40g) in concentrated hydrochloric acid (500ml) at 80°C. was treated dropwise with aqueous hydrogen peroxide solution (200ml;15%w/w), keeping the temperature between 80°C. and 85°C. The solution was then cooled and basified by dropwise treatment with aqueous sodium hydroxide solution (50%w/w), keeping the temperature below 15°C. The resulting white flocculent precipitate was recrystallised from toluene, to give 4-amino-3,5-dichloropyridine (61.5g), m.p. 161.5-162.5°C.

### REFERENCE EXAMPLE 5

A solution of 4-aminopyridine (47g) in concentrated hydrochloric acid (355ml) was treated portionwise at 80°C with an aqueous solution of sodium hypochlorite (550ml;15%w/v). The mixture was cooled to 30°C and basified by treatment with aqueous sodium hydroxide solution (300ml;35%w/v) during 20 minutes. The mixture was stirred and cooled for a further 30 minutes and then it was filtered. The solid was washed well with water and dried at 60°C, to give 4-amino-3,5-dichloropyridine (69.5g).

### REFERENCE EXAMPLE 6

A solution of 3-cyclopentyloxy-4-methoxybenzaldehyde (66g) and sulphamic acid (39.6g) in glacial acetic acid (500ml) was treated dropwise during 1 hour with a solution of sodium chlorite (35g) in water (150ml). The mixture was stirred at 20°C during 1 hour and then it was treated with water (500ml) dropwise during 30 minutes. The resulting solid was filtered, washed with water and dried, to give 3-cyclopentyloxy-4-methoxybenzoic acid (60.9g) in the form of white crystals [Elemental analysis:- C,65.8; H,6.7%; calculated:- C,66.1;H,6.8%].

### REFERENCE EXAMPLE 7

A solution of triphenylphosphine (17.5g) in dry tetrahydrofuran (50ml) under nitrogen was treated with a solution of diisopropyl azodicarboxylate (13.5g) in dry tetrahydrofuran (50ml). The solution was stirred was treated with a solution of endo-8,9,10-trinorborneol (5.0g) in dry tetrahydrofuran (50ml) followed by a solution of 3-hydroxy-4-methoxybenzaldehyde (10.2g) in dry tetrahydrofuran (50ml). The solution was heated at reflux for 15 hours, cooled, poured into water (600ml), and extracted with diethyl ether (300ml). The extract was washed with water (100ml), with aqueous sodium hydroxide solution (2x100ml;1M) and with water (2x100ml), dried over magnesium sulphate and evaporated, to give an oil, which was subjected to flash chromatography on silica gel, eluting with a mixture of pentane and ethyl acetate (95:5v/v) to give 3-(exo-8,9,10-trinorbornyl-2-oxy)-4-methoxybenzaldehyde (8.2g), m.p. 56-61°C.

### REFERENCE EXAMPLE 8

A stirred suspension of 3-hydroxy-4-methoxybenzaldehyde (50g) in water (200ml) at between 0 and 5°C was treated dropwise with an aqueous solution of sodium hydroxide (200ml;20%w/v), followed at between 0 and 5°C by benzoyl chloride (38ml). The reaction mixture was stirred at between 0 and 5°C for 1 hour and then it was allowed to warm to room temperature and was stirred for a further period of 2 hours. The resulting solution was extracted with dichloromethane (2x200ml) and the combined extract was washed with water (200ml), dried over magnesium sulphate and concentrated, to give 2-methoxy-5-formylphenyl benzoate (35.2g), m.p.70-72°C.

### REFERENCE EXAMPLE 9

A stirred solution of potassium permanganate (28g) in acetone (200ml) was treated with 2-methoxy-5-formylphenyl benzoate (35.2g; prepared as described in Reference Example 8), and the resulting vigorously reacting mixture was cooled in an ice bath. It was then stirred at room temperature for 3 hours. The mixture was then concentrated and the residue was treated with saturated aqueous sodium metabisulphite solution (300ml). The resulting white solid was filtered off, washed well with water (200ml), and dried, to give 3-benzoyloxy-4-methoxybenzoic acid (29.3g), m.p. 180-183°C.

### REFERENCE EXAMPLE 10

A solution of 3-benzoyloxy-4-methoxybenzoic acid (29.3g; prepared as described in Reference Example 9) in toluene (300ml) was treated with thionyl chloride (30ml) and heated on the steam bath for 6 hours. It was then cooled, filtered and concentrated, to give 3-benzoyloxy-4-methoxybenzoyl chloride (28.7g), m.p. 120-122°C.

### REFERENCE EXAMPLE 11

By proceeding in a manner similar to that described in Example 8, but using 3-benzoyloxy-4-methoxybenzoyl chloride (prepared as described in Reference Example 10) and 4-amino-3,5-dichloropyridine (prepared as described in Reference Example 4) as starting materials, there was prepared N-(3,5-dichloropyrid-4-yl)-3-benzoyloxy-4-methoxybenzamide, m.p. 191-192°C.

### REFERENCE EXAMPLE 12

A solution of N-(3,5-dichloropyrid-4-yl)-3-benzoyloxy-4-methoxybenzamide (13.4g; prepared as described in Reference Example 11) in methanol (160ml) and water (60ml) was treated with anhydrous potassium carbonate (18g), and stirred overnight at room temperature. It was then brought to pH7 by treatment with dilute hydrochloric acid (2N), and concentrated. The residue was treated with water (100ml) and filtered, and the resulting solid was dried, to give N-(3,5-dichloropyrid-4-yl)-3-hydroxy-4-methoxybenzamide (8.8g), m.p. 227-228°C.

### REFERENCE EXAMPLE 13

By proceeding in a manner similar to that described in Reference Example 2, but using the appropriate quantities of 3-(exo-8,9,10-trinorbornyl-2-oxy)-4-methoxybenzaldehyde (prepared as described in Reference Example 7) and (R)-3-(exo-8,9,10-trinorbornyl-2-oxy)-4-methoxybenzaldehyde and (S)-3-(exo-8,9,10-trinorbornyl-2-oxy)-4-methoxybenzaldehyde (similarly prepared from (R)-endo-8,9,10-trinorborneol and (S)-endo-8,9,10-trinorborneol or as described in the specification of European Patent Publication No. 0428302A2] there were prepared:-
3-(exo)-8,9,10-trinorbornyl-2-oxy-4-methoxybenzoic acid, m.p. 155-156°C;
(R)-3-(exo)-8,9,10-trinorbornyl-2-oxy-4-methoxybenzoic acid, m.p. 155-156°C; and
(S)-3-(exo)-8,9,10-trinorbornyl-2-oxy-4-methoxybenzoic acid, m.p. 155-156°C.

### REFERENCE EXAMPLE 14

By proceeding in a manner similar to that described in Reference Example 3, but using the appropriate quantities of the corresponding benzoic acid derivatives, prepared as described hereinbefore in Reference Example 13, there were prepared:-
3-(exo)-8,9,10-trinorbornyl-2-oxy-4-methoxybenzoyl chloride;
(R)-3-(exo)-8,9,10-trinorbornyl-2-oxy-4-methoxybenzoyl chloride; and
(S)-3-(exo)-8,9,10-trinorbornyl-2-oxy-4-methoxybenzoyl chloride; each in the form of oils.

The present invention also includes within its scope pharmaceutical formulations which comprise at least one of the compounds of formula I or a pharmaceutically acceptable salt thereof in association with a pharmaceutically acceptable carrier or coating.

In clinical practice compounds of the present invention may be administered parenterally, rectally or orally, but they are preferably administered by inhalation.

Suitable compositions containing compounds of the invention and pharmaceutically acceptable salts thereof may be prepared by conventional means. For example, compounds of the invention and pharmaceutically acceptable salts thereof may be dissolved or suspended in a suitable carrier for use in a nebuliser or a suspension or solution aerosol, or may be absorbed or adsorbed onto a suitable solid carrier for use in a dry powder inhaler.

Solid compositions for oral administration include compressed tablets, pills, powders and granules. In such solid compositions, one or more of the active compounds is, or are, admixed with at least one inert diluent such as starch, sucrose or lactose. The compositions may also comprise, as is normal practice, additional substances other than inert diluents, e.g. lubricating agents, such as magnesium stearate.

Liquid compositions for oral administration include pharmaceutically acceptable emulsions, solutions, suspensions, syrups and elixirs containing inert diluents commonly used in the art such as water and liquid paraffin. Besides inert diluents such compositions may comprise adjuvants, such as wetting and suspending agents, and sweetening, flavouring, perfuming and preserving agents. The compositions according to the invention for oral administration also include capsules of absorbable material such as gelatin, containing one or more of the active substances with or without the addition of diluents or excipients.

Compositions according to the invention for parenteral administration include sterile aqueous, aqueous-organic, and organic solutions, suspensions and emulsions. Examples of organic solvents or suspending media are propylene glycol, polyethylene glycol, vegetable oils such as olive oil and injectable organic esters such as ethyl oleate. The compositions may also contain adjuvants such as stabilising, preserving, wetting, emulsifying and dispersing agents. They may be sterilised, for example, by filtration through a bacteria-retaining filter, by incorporation in the compositions of sterilising agents, by irradiation or by heating. They may also be manufactured in the form of sterile solid compositions, which can be dissolved in sterile water or some other sterile injectable medium immediately before use.

Solid compositions for rectal administration include suppositories formulated in accordance with known methods and containing at least one compound of formula I.

The percentage of active ingredient in the compositions of the invention may be varied, it being necessary that it should constitute a proportion such that a suitable dosage shall be obtained. Obviously, several unit dosage forms may be administered at about the same time. The dose employed will be determined by the physician, and depends upon the desired therapeutic effect, the route of administration and the duration of the treatment, and the condition of the patient. In the adult, the doses are generally from about 0.001 to about 50, preferably about 0.001 to about 5, mg/kg body weight per day by inhalation.

The following Composition Examples illustrate pharmaceutical compositions according to the present invention.

### COMPOSITION EXAMPLE 1

N-(2,6-difluorophenyl)-3-cyclopentyloxy-4-methoxybenzamide (1.0g) (mean particle size 3.5 microns) and lactose (mean particle size 72 microns) were blended together for 30 minutes in a mechanical shaker/mixer. The resulting blend was filled, to a fill weight of 25mg, into No.3 hard gelatine capsules, to give a product suitable for use, for example, with a dry powder inhaler.

### COMPOSITION EXAMPLE 2

N-(3,5-dichloropyrid-4-yl)-3-cyclopentyloxy-4-methoxybenzamide (1.0g) (mean particle size 3.5 microns) and lactose (mean particle size 72 microns) were blended together for 30 minutes in a mechanical shaker/mixer. The resulting blend was filled, to a fill weight of 25mg, into No.3 hard gelatine capsules, to give a product suitable for use, for example, with a dry powder inhaler.

## Claims

1. A benzamide derivative of the general formula (I): 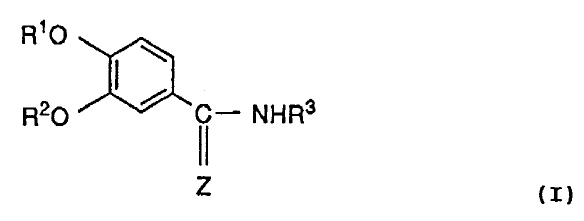 wherein R¹ represents a straight- or branched-chain alkyl group containing up to 4 carbon atoms, R² represents a straight- or branched-chain alkyl group containing from 2 to 15 carbon atoms or a mono-, bi- or tricycloalkyl group containing up to 10 carbon atoms, R³ represents an optionally substituted phenyl, naphthyl or heterocyclyl group, and Z represents an oxygen or sulphur atom, and when said heterocyclyl groups contain one or more nitrogen ring atoms, N-oxides thereof, and pharmaceutically acceptable salts thereof.

2. A compound according to claim 1 with the proviso that, when R³ represents an unsubstituted pyrid-4-yl group and Z represents an oxygen atom, R² does not represent an alkyl group containing 2, 3 or 4 carbon atoms.

3. A compound according to claim 1 or 3 wherein the heterocyclyl group is a 5-, 6- or 7-membered heterocyclyl group containing one or more hetero atoms selected from oxygen, sulphur and nitrogen atoms, and the optional substituents on phenyl, naphthyl or heterocyclyl being one or more substituents selected from halogen atoms, alkyl groups which may carry one or more halogen atoms, and from aryl, arylalkyl, alkoxy, aryloxy, alkylthio, arylthio, alkoxycarbonyl, aryloxycarbonyl, alkanoyl, aroyl, alkylsulphonyl, arylsulphonyl, alkylsulphinyl, arylsulphinyl, hydroxy, hydroxyalkyl, formyl, alkanoylamino, aroylamino, cyano and nitro groups, and from amino, carbamoyl and sulphamoyl groups which themselves may each carry one or two alkyl substituents, wherein all aryl groups and moieties, unless otherwise indicated, are selected from phenyl and naphtbyl groups optionally substituted by one or more substituents selected from halogen atoms and alkyl and alkoxy groups, and wherein all alkyl groups and moieties, unless otherwise indicated, are straight- or branched-chain and contain up to 4 carbon atoms.

4. A compound according to claim 1, 2 or 3 wherein R² represents a straight- or branched-chain alkyl group containing from 2 to 12 carbon atoms,

5. A compound according to claim 1, 2 or 3 wherein at least one of the symbols has a value selected from the following:-
(i) R¹ represents a methyl group; and/or
(ii) R² represents a propyl, butyl, nonyl, dodecyl, cyclohexyl, 8,9,10-trinorbornyl or cyclopentyl group; and/or
(iii) R³ represents an optionally substituted pyrazinyl, pyrimidinyl, isoxazolyl, or pyridyl group, or an N-oxide thereof, or an optionally substituted phenyl group;
the other symbols being as defined in claim 1, 2 or 3.

6. A compound according to claim 1, 2, 3 or 5 wherein R² represents a cyclopentyl group and R³ represents an optionally substituted pyridyl group.

7. A compound according to any one of claims 1 to 5 wherein R³ represents a phenyl group which is substituted in the 2-position or in the 2- and 6-positions.

8. A compound according to any one of claims 1 to 6 wherein R³ represents a heterocyclyl group substituted on one or both of the positions next to the point of attachment to the rest of the molecule.

9. A compound according to claim 1, 2 or 3 which is
N-(2,6-difluorophenyl)-3-cyclopentyloxy-4-methoxybenzamide,
N-(2-chloro-6-fluorophenyl)-3-cyclopentyloxy-4-methoxybenzamide,
N-(2-trifluoromethylphenyl)-3-cyclopentyloxy-4-methoxybenzamide,
N-(2,4,6-trichlorophenyl)-3-cyclopentyloxy-4-methoxybenzamide,
N-(2,6-dibromophenyl)-3-cyclopentyloxy-4-methoxybenzamide,
N-(2-chloro-6-methylphenyl)-3-cyclopentyloxy-4-methoxybenzamide,
N-(2,6-dichlorophenyl)-3-cyclopentyloxy-4-methoxybenzamide,
N-(2-fluorophenyl)-3-cyclopentyloxy-4-methoxybenzamide,
N-phenyl-3-cyclopentyloxy-4-methoxybenzamide,
N-(2-methoxyphenyl)-3-cyclopentyloxy-4-methoxybenzamide,
N-(2-chlorophenyl)-3-cyclopentyloxy-4-methoxybenzamide,
N-(3-chlorophenyl)-3-cyclopentyloxy-4-methoxybenzamide,
N-(4-methoxyphenyl)-3-cyclopentyloxy-4-methoxybenzamide,
N-(2,6-dimethylphenyl)-3-cyclopentyloxy-4-methoxybenzamide,
N-(2-methylthiophenyl)-3-cyclopentyloxy-4-methoxybenzamide,
N-(2-bromophenyl)-3-cyclopentyloxy-4-methoxybenzamide,
N-(2-methoxycarbonylphenyl)-3-cyclopentyloxy-4-methoxybenzamide,
N-(2-aminosulfonylphenyl)-3-cyclopentyloxy-4-methoxybenzamide,
N-(2-benzoylphenyl)-3-cyclopentyloxy-4-methoxybenzamide,
N-(2-cyanophenyl)-3-cyclopentyloxy-4-methoxybenzamide,
N-(2,5-dichlorophenyl)-3-cyclopentyloxy-4-methoxybenzamide,
N-(3-methylphenyl)-3-cyclopentyloxy-4-methoxybenzamide,
N-(2-nitrophenyl)-3-cyclopentyloxy-4-methoxybenzamide,
N-(2-dimethylaminophenyl)-3-cyclopentyloxy-4-methoxybenzamide,
N-(2-acetylphenyl)-3-cyclopentyloxy-4-methoxybenzamide,
N-(2-hydroxyphenyl)-3-cyclopentyloxy-4-methoxybenzamide,
N-(2-methylsulfonylphenyl)-3-cyclopentyloxy-4-methoxybenzamide,
N-(2,6-difluorophenyl)-3-cyclohexyloxy-4-methoxybenzamide,
N-(2,6-difluorophenyl)-3-butoxy-4-methoxybenzamide,
N-(2,6-difluorophenyl)-3-propoxy-4-methoxybenzamide,
N-(2-chlorophenyl)-3-cyclopentyloxy-4-methoxy(thiobenzamide),
N-(4-chloropyrid-3-yl)-3-cyclopentyloxy-4-methoxybenzamide,
N-pyrid-2-yl-3-cyclopentyloxy-4-methoxybenzamide,
N-pyrazin-2-yl-3-cyclopentyloxy-4-methoxybenzamide,
N-pyrimidin-2-yl-3-cyclopentyloxy-4-methoxybenzamide,
N-(3-methylpyrid-2-yl)-3-cyclopentyloxy-4-methoxybenzamide,
N-pyrid-3-yl-3-cyclopentyloxy-4-methoxybenzamide,
N-(3-chloropyrid-2-yl)-3-cyclopentyloxy-4-methoxybenzamide,
N-(3-chloropyrid-4-yl)-3-cyclopentyloxy-4-methoxybenzamide,
N-pyrid-4-yl)-3-cyclopentyloxy-4-methoxybenzamide,
N-(3,5-dichloropyrid-4-yl)-3-cyclopentyloxy-4-methoxybenzamide,
N-(3,5-dimethylisoxazol-4-yl)-3-cyclopentyloxy-4-methoxybenzamide,
N-(4,6-dichloropyrimid-5-yl)-3-cyclopentyloxy-4-methoxybenzamide,
N-(4-nitrophenyl)-3-cyclopentyloxy-4-methoxybenzamide,
N-(2,3,5,6-tetrafluoropyrid-4-yl)-3-cyclopentyloxy-4-methoxybenzamide,
N-(3,5-dichloro-2,6-difluoropyrid-4-yl)-3-cyclopentyloxyd-4-methoxybenzamide,
N-(2,4,6-trifluorophenyl)-3-cyclopentyloxy-4-methoxybenzamide,
3,5-dichloro-4-(3-cyclopentyloxy-4-methoxybenzamido)pyridine-N-oxide,
N-(3,5-dichloropyrid-4-yl)-3-(exo-8,9,10-trinorbornyl-2-oxy)-4-methoxybenzamide,
N-(3,5-dichloropyrid-4-yl)-3-cyclohexyloxy-4-methoxybenzamide,
N-(3,5-dibromopyrid-4-yl)-3-cyclopentyloxy-4-methoxybenzamide,
N-(3,5-dichloropyrid-4-yl)-3-butoxy-4-methoxybenzamide,
N-(3-methyl-5-bromoisothiazol-4-yl)-3-cyclopentyloxy-4-methoxybenzamide,
N-(3,5-dimethylisothiazol-4-yl)-3-cyclopentyloxy-4-methoxybenzamide,
N-(3,5-dimethylpyrid-4-yl)-3-cyclopentyloxy-4-methoxybenzamide,
N-(5-cyano-3-methylisothiazol-4-yl)-3-cyclopentyloxy-4-methoxybenzamide,
N-(3,5-dichloropyrid-4-yl)-3-cyclopentyloxy-4-methoxy(thiobenzamide),
N-(2,6-dichloro-4-methoxyphenyl)-3-cyclopentyloxy-4-methoxybenzamide,
N-(2,6-dichloro-4-cyanophenyl)-3-cyclopentyloxy-4-methoxybenzamide,
N-(2,6-dichloro-4-carbamoylphenyl)-3-cyclopentyloxy-4-methoxybenzamide,
N-(2,6-dichloro-4-aminophenyl)-3-cyclopentyloxy-4-methoxybenzamide,
N-(3-chloro-2,5,6-trifluoropyrid-4-yl)-3-cyclopentyloxy-4-methoxybenzamide,
N-(3,5-dibromopyrid-4-yl)-3-butoxy-4-methoxybenzamide,
N-(2,6-dichloro-4-methoxycarbonylphenyl)-3-cyclopantyloxy-4-methoxybenzamide,
N-(4-acetylamino-2,6-dichlorophenyl)-3-cyclopentyloxy-4-methoxybenzamide,
N-(3,5-dichloropyrid-4-yl)-3-nonyloxy-4-methoxybenzamide,
N-(2,6-dichloro-4-formylphenyl)-3-cyclopentyloxy-4-methoxybenzamide,
N-(2,6-dichlorophenyl)-3-(exo-8,9,10-trinorbornyl-2-oxy)-4-methoxybenzamide,
N-(2,3,5-trifluoropyrid-4-yl)-3-cyclopentyloxy-4-methoxybenzamide,
sodium salt of N-(3,5-dichloropyrid-4-yl)-3-cyclopentyloxy-4-methoxybenzamide,
N-(2,6-dichloro-4-ethoxycarbonylphenyl)-3-cyclopentyloxy-4-methoxybenzamide,
N-(2,6-dichloro-4-hydroxymethylphenyl)-3-cyclopentyloxy-4-methoxybenzamide,
N-(3,5-dichloropyrid-4-yl)-3-dodecyloxy-4-methoxybenzamide,
(R)-N-(3,5-dichloropyrid-4-yl)-3-(exo-8,9,10-trinorbornyl-2-oxy)-4-methoxybenzamide,
(S)-N-(3,5-dichloropyrid-4-yl)-3-(exo-8,9,10-trinorbornyl-2-oxy)-4-methoxybenzamide,
N-(2,6-dichloro-4-nitrophenyl)-3-cyclopentyloxy-4-methoxybenzamide
or a pharmaceutically acceptable salt thereof,

10. The compound according to claim 1, 2, 3, 5, 6, 8 or 9 which is N-(3,5-dichloropyrid-4-yl)-3-cyclopentyloxy-4-methoxybenzamide.

11. A process for the preparation of a compound according to any one of the preceding claims which comprises
(a) the reaction of a compound of the general formula (II) : 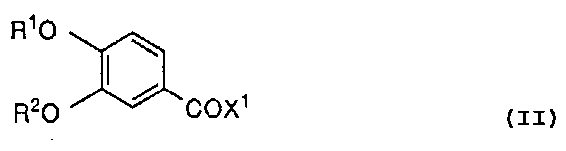 wherein R¹ and R² are as defined in claim 1 and X¹ represents a halogen atom with a compound of the general formula (III) :
NH₂R³ (III)
wherein R³ is as defined in claim 1, or
(b) the reaction of a compound of the general formula (IV): 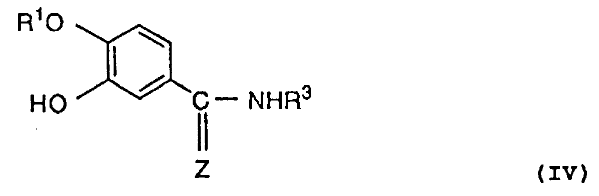 wherein R¹, R² and Z are as defined in claim 1 with a compound of the general formula (V):
R²X² (V)
wherein R² is as defined in claim 1 and X² represents a halogen atom;
optionally followed by the conversion of a compound of general formula (I) thus obtained into another compound of general formula (I);
and optionally converting the compound of general formula (I) thus obtained into a salt thereof.

12. A pharmaceutical composition which comprises a benzamide derivative of general formula (I) as defined in any one of claims 1 to 10 or a pharmaceutically acceptable salt thereof in association with a pharmaceutically acceptable carrier or coating.

13. Use of a benzamide derivative as defined in any one of claims 1 to 10 or a pharmaceutically acceptable salt thereof for the preparation of a medicine useful for the treatment of a human or animal patient suffering from, or subject to, a condition which can be ameliorated by the administration of an inhibitor of cyclic AMP phosphodiesterase.

## Patentansprüche

1. Benzamidderivat der allgemeinen Formel (I) 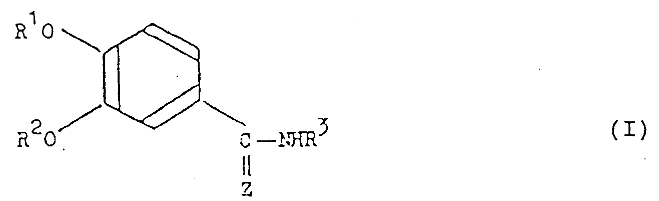 Worin R¹ eine geradkettige oder verzweigte Alkylgruppe mit bis zu 4 Kohlenstoffatomen bedeutet, R² für eine geradkettige oder verzweigte Alkylgruppe mit 2 bis 15 Kohlenstoffatomen oder eine Mono-, Bi- oder Tricycloalkylgruppe mit bis zu 10 Kohlenstoffatomen steht, R³ eine gegebenenfalls substituierte Phenyl-, Naphthyl- oder Heterocyclylgruppe wiedergibt und Z ein Sauerstoff- oder Schwefelatom bedeutet, und wenn die Heterocyclylgruppen ein oder mehrere Stickstoffringatome enthalten, deren N-Oxide und pharmazeutisch verträgliche Salze hiervon.

2. Verbindung gemäß Anspruch 1 mit der Maßgabe, daß, wenn R³ eine unsubstituierte Pyrid-4-yl-gruppe ist und Z ein Sauerstoffatom bedeutet, R² keine Alkylgruppe mit 2, 3 oder 4 Kohlenstoffatomen darstellt.

3. Verbindung gemäß Anspruch 1 oder 2, worin die Heterocyclylgruppe eine 5-, 6- oder 7-gliedrige Heterocyclylgruppe mit einem oder mehreren Heteroatomen, ausgewählt unter Sauerstoff-, Schwefel- und Stickstoffatomen, ist und die etwaigen Substituenten an Phenyl, Naphthyl oder Heterocyclyl ein oder mehrere Substituenten sind, ausgewählt unter Halogenatomen, Alkylgruppen, die ein oder mehrere Halogenatome aufweisen können, und unter Aryl-, Aralkyl-, Alkoxy-, Aryloxy-, Alkylthio-, Arylthio-, Alkoxycarbonyl-, Aryloxycarbonyl-, Alkanoyl-, Aroyl-, Alkylsulfonyl-, Arylsulfonyl-, Alkylsulfinyl-, Arylsulfinyl-, Hydroxy-, Hydroxyalkyl-, Formyl-, Alkanoylamino-, Aroylamino-, Cyano- und Nitrogruppen und unter Amino-, Carbamoyl- und Sulfamoylgruppen, die ihrerseits jeweils einen oder zwei Alkyl-substituenten aufweisen können, wobei sämtliche Arylgruppen und Teile, sofern nicht anders angegeben, ausgewählt sindunter Phenyl- und Naphthylgruppen, welche gegebenenfalls substituiert sind durch einen oder mehrere Substituenten, ausgewählt unter Halogenatomen und Alkyl- und Alkoxygruppen, und worin sämtliche Alkylgruppen und Teile, sofern nicht anders angegeben, geradkettig oder verzweigt sind und bis zu 4 Kohlenstoffatome enthalten.

4. Verbindung gemäß Anspruch 1, 2 oder 3, worin R² für eine geradkettige oder verzweigte Alkylgruppe mit 2 bis 12 Kohlenstoffatomen steht.

5. Verbindung gemäß Anspruch 1, 2 oder 3, worin zumindest eines der Symbole eine Bedeutung besitzt, ausgewählt unter den folgenden:
(i) R¹ bedeutet eine Methylgruppe; und/oder
(ii) R² bedeutet eine Propyl-, Butyl-, Nonyl-, Dodecyl-, Cyclohexyl-, 8,9,10-Trinorbornyl- oder Cyclopentylgruppe; und/oder
(iii) R³ bedeutet eine gegebenenfalls substituierte Pyrazinyl-, Pyrimidinyl-, Isoxazolyl- oder Pyridylgruppe oder ein N-Oxid hiervon oder eine gegebenenfalls substituierte Phenylgruppe; wobei die anderen Symbole wie in Anspruch 1, 2 oder 3 definiert sind.

6. Verbindung gemäß Anspruch 1, 2, 3 oder 5, worin R² für eine Cyclopentylgruppe steht und R³ eine gegebenenfalls substituierte Pyridylgruppe bedeutet.

7. Verbindung gemäß einem der Ansprüche 1 bis 5, worin R³ eine Phenylgruppe ist, die in der 2-Stellung oder in der 2- und 6-Stellung substituiert ist.

8. Verbindung gemäß einem der Ansprüche 1 bis 6, Worin R³ eine Heterocyclylgruppe bedeutet, die an einer oder beiden der an die Verknüpfungsstelle zu dem Rest des Moleküls angrenzenden Positionen substituiert ist.

9. Verbindung gemäß Anspruch 1, 2 oder 3, nämlich N-(2,6-Difluorphenyl)-3-cyclopentyloxy-4-methoxybenzamid,
N-(2-Chlor-6-fluorphenyl)-3-cyclopentyloxy-4-methoxybenzamid,
N-(2-Trifluormethylphenyl)-3-cyclopentyloxy-4-methoxybenzamid,
N-(2,4,6-Trichlorphenyl)-3-cyclopentyloxy-4-methoxybenzamid,
N-(2,6-Dibromphenyl)-3-cyclopentyloxy-4-methoxybenzamid,
N-(2-Chlor-6-methylphenyl)-3-cyclopentyloxy-4-methoxybenzamid,
N-(2,6-Dichlorphenyl)-3-cyclopentyloxy-4-methoxybenzamid,
N-(2-Fluorphenyl)-3-cyclopentyloxy-4-methoxybenzamid,
N-Phenyl-3-cyclopentyloxy-4-methoxybenzamid,
N-(2-Methoxyphenyl)-3-cyclopentyloxy-4-methoxybenzamid,
N-(2-Chlorphenyl)-3-cyclopentyloxy-4-methoxybenzamid,
N-(3-Chlorphenyl)-3-cyclopentyloxy-4-methoxybenzamid,
N-(4-Methoxyphenyl)-3-cyclopentyloxy-4-methoxybenzamid,
N-(2,6-Dimethylphenyl)-3-cyclopentyloxy-4-methoxybenzamid,
N-(Methylthiophenyl)-3-cyclopentyloxy-4-methoxybenzamid,
N-(2-Bromphenyl)-3-cyclopentyloxy-4-methoxybenzamid,
N-(2-Methoxycarbonylphenyl-3-cyclopentyloxy-4-methoxybenzamid,
N-(2-Aminosulfonylphenyl)-3-cyclopentyloxy-4-methoxybenzamid,
N-(2-Benzoylphenyl)-3-cyclopentyloxy-4-methoxybenzamid,
N-(2-Cyanophenyl)-3-cyclopentyloxy-4-methoxybenzamid,
N-(2,5-Dichlorphenyl)-3-cyclopentyloxy-4-methoxybenzamid,
N-(3-Methylphenyl ) -3-cyclopentyloxy-4-methoxybenzamid,
N-(2-Nitrophenyl)-3-cyclopentyloxy-4-methoxybenzamid,
N-(2-Dimethylaminophenyl)-3-cyclopentyloxy-4-methoxybenzamid,
N-(2-Acetylphenyl)-3-cyclopentyloxy-4-methoxybenzamid,
N-(2-Hydroxyphenyl)-3-cyclopentyloxy-4-methoxybenzamid,
N-(2-Methylsulfonylphenyl)-3-cyclopentyloxy-4-methoxybenzamid,
N-(2,6-Difluorphenyl)-3-cyclopentyloxy-4-methoxybenzamid,
N-(2,6-Difluorphenyl)-3-butoxy-4-methoxybenzamid,
N-(2,6-Difluorphenyl)-3-propoxy-4-methoxybenzamid,
N-(2-Chloryhenyl)-3-cyclopentyloxy-4-methoxy-(thiobenzamid),
N-(4-Chlorpyrid-3-yl)-3-cyclopentyloxy-4-methoxybenzamid,
N-Pyrid-2-yl-3-cyclopentyloxy-4-methoxybenzamid,
N-Pyrazin-2-yl-3-cyclopentyloxy-4-methoxybenzamid,
N-Pyrimidin-2-yl-3-cyclopentyloxy-4-methoxybenzamid,
N-(3-Methylpyrid-2-yl)-3-cyclopentyloxy-4-methoxybenzamid,
N-Pyrid-3-yl-3-cyclopentyloxy-4-methoxybenzamid,
N-(3-Chlorpyrid-2-yl)-3-cyclopentyloxy-4-methoxybenzamid,
N-(3-Chlorpyrid-4-yl)-3-cyclopentyloxy-4-methoxybenzamid,
N-Pyrid-4-yl-3-cyclopentyloxy-4-methoxybenzamid,
N-(3,5-Dichlorpyrid-4-yl)-3-cyclopentyloxy-4-methoxybenzamid,
N-(3,5-Dimethylisoxazol-4-yl)-3-cyclopentyloxy-4-methoxybenzamid,
N-(4,6-Dichlorpyrimid-5-yl)-3-cyclopentyloxy-4-methoxybenzamid,
N-(4-Nitrophenyl)-3-cyclopentyloxy-4-methoxybenzamid,
N-(2,3,5,6-Tetrafluorpyrid-4-yl)-3-cyclopentyloxy-4-methoxybenzamid,
N-(3,5-Dichlor-2,6-difluorpyrid-4-yl)-3-cyclopentyloxy-4-methoxybenzamid,
N-(2,4,6-Trifluorphenyl)-3-cyclopentyloxy-4-methoxybenzamid,
3,5-Dichlor-4-(3-cyclopentyloxy-4-methoxybenzamido)-pyridin-N-oxid,
N-(3,5-Dichlorpyrid-4-yl)-3-(exo-8,9,10-trinorbornyl-2-oxy)-4-methoxybenzamid,
N-(3,5-Dichlorpyrid-4-yl)-3-cyclopentyloxy-4-methoxybenzamid,
N-(3,5-Dibrompyrid-4-yl)-3-cyclopentyloxy-4-methoxybenzamid,
N-(3,5-Dichlorpyrid-4-yl)-3-butoxy-4-methoxybenzamid,
N-(3-Methyl-5-bromisothiazol-4-yl)-3-cyclopentyloxy-4-methoxybenzamid,
N-(3,5-Dimethylisothiazol-4-yl)-3-cyclopentyloxy-4-methoxybenzamid,
N-(3,5-Dimethylpyrid-4-yl)-3-cyclopentyloxy-4-methoxybenzamid,
N-(5-Cyano-3-methylisothiazol-4-yl)-3-cyclopentyloxy-4-methoxybenzamid,
N-(3,5-Dichlorpyrid-4-yl)-3-cyclopentyloxy-4-methoxy-(thiobenzamid),
N-(2,6-Dichlor-4-methoxyphenyl)-3-cyclopentyloxy-4-methoxybenzamid,
N-(2,6-Dichlor-4-cyanophenyl)-3-cyclopentyloxy-4-methoxybenzamid,
N-(2,6-Dichlor-4-carbamoylphenyl)-3-cyclopentyloxy-4-methoxybenzamid,
N-(2,6-Dichlor-4-aminophenyl)-3-cyclopentyloxy-4-methoxybenzamid,
N-(3-Chlor-2,5,6-trifluorpyrid-4-yl)-3-cyclopentyloxy-4-methoxybenzamid,
N-(3,5-Dibrompyrid-4-yl)-3-butoxy-4-methoxybenzamid,
N-(2,6-Dichlor-4-methoxycarbonylphenyl)-3-cyclopentyloxy-4-methoxybenzamid,
N-(4-Acetylamino-2,6-dichlorphenyl)-3-cyclopentyloxy-4-methoxybenzamid,
N-(3,5-Dichlorpyrid-4-yl)-3-nonyloxy-4-methoxybenzamid,
N-(2,6-Dichlor-4-formylphenyl)-3-cyclopentyloxy-4-methoxybenzamid,
N-(2,6-Dichlorphenyl)-3-(exo-8,9,10-trinorbornyl-2-oxy)-4-methoxybenzamid,
N-(2,3,5-Trifluorpyrid-4-yl)-3-cyclopentyloxy-4-methoxybenzamid,
Natriumsalz von N-(3,5-Dichlorpyrid-4-yl)-3-cyclopentyloxy-4-methoxybenzamid,
N-(2,6-Dichlor-4-ethoxycarbonylphenyl)-3-cyclopentyloxy-4-methoxybenzamid,
N-(2,6-Dichlor-4-hydroxymethylphenyl)-3-cyclopentyloxy-4-methoxybenzamid,
N-(3,5-Dichlorpyrid-4-yl)-3-dodecyloxy-4-methoxybenzamid,
(R)-N-(3,5-Dichlorpyrid-4-yl)-3-(exo-8,9,10-trinorbornyl-2-oxy)-4-methoxybenzamid,
(S)-N-(3,5-Dichlorpyrid-4-yl)-3-(exo-8,9,10-trinorbornyl-2-oxy)-4-methoxybenzamid,
N-(2,6-Dichlor-4-nitrophenyl)-3-cyclopentyloxy-4-methoxybenzamid
oder ein pharmazeutisch verträgliches Salz hiervon.

10. Verbindung gemaß Anspruch 1, 2, 3, 5, 6, 8 oder 9, nämlich N-(3,5-Dichlorpyrid-4-yl)-3-cyclopentyloxy-4-methoxybenzamid.

11. Verfahren zur Herstellung einer Verbindung gemäß einem der vorhergehenden Ansprüche, umfassend
(a) die Umsetzung einer Verbindung der allgemeinen Formel (II) 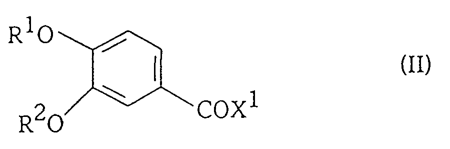 worin R¹ und R² wie in Anspruch 1 definiert sind und X¹ ein Halogenatom bedeutet, mit einer Verbindung der allgemeinen Formel (III)
NH₂R³ (III)
worin R³ wie in Anspruch 1 definiert ist; oder
(b) die Umsetzung einer Verbindung der allgemeinen Formel (IV) 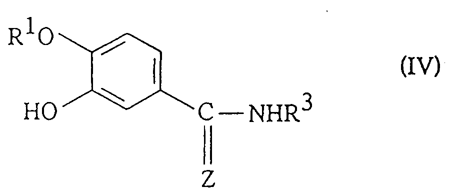 Worin R¹, R² und Z Wie in Anspruch 1 definiert sind, mit einer Verbindung der allgemeinen Formel (V)
R²X² (V)
worin R² wie in Anspruch 1 definiert ist und X² ein Halogenatom bedeutet,
woran sich gegebenenfalls anschließt die Umwandlung einer so erhaltenen Verbindung der allgemeinen Formel (I) in eine andere Verbindung der allgemeinen Formel (I); und gegebenenfalls Umwandlung der so erhaltenen Verbindung der allgemeinen Formel (I) in ein Salz hiervon.

12. Pharmazeutische Zusammensetzung, die ein Benzamidderivat der allgemeinen Formel (I), wie in einem der Ansprüche 1 bis 10 definiert, oder ein pharmazeutisch verträgliches Salz hiervon zusammen mit einem pharmazeutisch verträglichen Träger oder Überzug umfaßt.

13. Verwendung eines Benzamidderivats, wie in einem der Ansprüche 1 bis 10 definiert oder eines pharmazeutisch annehmbaren Salzes hiervon, für die Herstellung eines Arzneimittels zur Behandlung eines menschlichen oder tierischen Patienten, der leidet an oder betroffen ist von einem Zustand, der durch Verabreichung eines Inhibitors von cyclischer AMP-Phosphodiesterase verbessert werden kann.

## Revendications

1. Dérivé de benzamide de formule générale (I) : 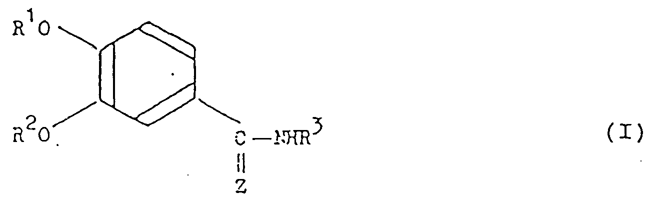 dans laquelle R¹ représente un groupe alkyle à chaîne linéaire ou ramifiée contenant jusqu'à 4 atomes de carbone, R² représente un groupe alkyle à chaîne linéaire ou ramifiée contenant de 2 à 15 atomes de carbone, ou un groupe mono-, bi- ou tricycloalkyle contenant jusqu'à 10 atomes de carbone, R³ représente un groupe phényle, naphtyle ou hétérocyclique éventuellement substitué et Z représente un atome d'oxygène ou de soufre, et lorsque lesdits groupes hétérocycliques contiennent un ou plusieurs atomes d'azote cycliques, leurs N-oxydes, et leurs sels pharmaceutiquement acceptables.

2. Composé selon la revendication 1, sous réserve que quand R³ représente un groupe pyridyl-4 non substitué et Z représente un atome d'oxygène, R² ne représente pas un groupe alkyl contenant 2, 3 ou 4 atomes de carbone.

3. Composé selon la revendication 1 ou 2, dans lequel le groupe hétérocyclique est un groupe hétérocyclique à 5-, 6- ou 7 chaÏnons contenant un ou plusieurs hétéroatomes choisis parmi les atomes d'oxygène, de soufre et d'azote, et les substituants facultatifs sur le groupe phényle, naphtyle ou hétérocyclique sont un ou plusieurs substituants choisis parmi les atomes d'halogène, les groupes alkyle qui peuvent porter un ou plusieurs atomes d'halogène, et parmi les groupes aryle, arylalkyle, alcoxy, aryloxy, alkylthio, arylthio, alcoxycarbonyle, aryloxycarbonyle, alcanoyle, aroyle, alkylsulfonyle, arylsulfonyle, alkylsulfinyle, arylsulfinyle, hydroxy, hydroxyalkyle, formyle, alcanoylamino, aroylamino, cyano et nitro, et parmi les groupes amino, carbamoyle et sulfamoyle qui peuvent eux-mêmes porter chacun un ou deux substituants alkyle, où tous les fragments et groupes aryle, sauf indication contraire, sont choisis parmi les groupes phényle et naphtyle éventuellement substitués par un ou plusieurs substituants choisis parmi les atomes d'halogène et les groupes alkyle et alcoxy et où tous les fragments et groupes alkyle, sauf indication contraire, sont à chaîne linéaire ou ramifiée et contiennent jusqu'à 4 atomes de carbone.

4. Composé selon la revendication 1, 2 ou 3, dans lequel R² représente un groupe alkyle à chaîne linéaire ou ramifiée contenant de 2 à 12 atomes de carbone.

5. Composé selon la revendication 1, 2 ou 3, dans lequel au moins un des symboles possède une valeur choisie parmi les suivantes :
(i) R¹ représente un groupe méthyle ;
(ii) R² représente un groupe propyle, butyle, nonyle, dodécyle, cyclohexyle, 8,9,10-trinorbornyle ou cyclopentyle ; et/ou
(iii) R³ représente un groupe pyrazinyle, pyrimidinyle, isoxazolyle ou pyridyle éventuellement substitué, ou un de leurs oxydes, ou un groupe phényle éventuellement substitué ;
les autres symboles étant tels que définis précédemment.

6. Composé selon la revendication 1, 2, 3 ou 5, dans lequel R² représente un groupe cyclopentyle et R³ représente un groupe pyridyle éventuellement substitué.

7. Composé selon l'une quelconque des revendications 1 à 5 précédentes, dans lequel R³ représente un groupe phényle qui est substitué en position 2 ou en positions 2 et 6.

8. Composé selon l'une quelconque des revendications 1 à 6, dans lequel R³ représente un groupe hétérocyclique substitué sur l'une des positions ou sur les deux positions voisines du point de fixation au reste de la molécule.

9. Composé selon la revendication 1, 2 ou 3, qui est le
N-(2,6-difluorophényl)-3-cyclopentyloxy-4-méthoxybenzamide,
N-(2-chloro-6-fluorophényl)-3-cyclopentyloxy-4-méthoxybenzamide,
N-(2-trifluorométhylphényl)-3-cyclopentyloxy-4-méthoxybenzamide,
N-(2,4,6-trifluorométhylphényl)-3-cyclopentyloxy-4-méthoxybenzamide,
N-(2,6-dibromophényl)-3-cyclopentyloxy-4-méthoxybenzamide,
N-(2-chloro-6-méthylphényl)-3-cyclopentyloxy-4-méthoxybenzamide
N-(2,6-dichlorophényl)-3-cyclopentyloxy-4-méthoxybenzamide
N-(2-fluorophényl)-3-cyclopentyloxy-4-méthoxybenzamide,
N-phényl-3-cyclopentyloxy-4-méthoxybenzamide,
N-(2-méthoxyphényl)-3-cyclopentyloxy-4-méthoxybenzamide,
N-(2-chlorophényl)-3-cyclopentyloxy-4-méthoxybenzamide,
N-(3-chlorophényl)-3-cyclopentyloxy-4-méthoxybenzamide,
N-(4-méthoxyphényl)-3-cyclopentyloxy-4-méthoxybenzamide,
N-(2,6-diméthylphényl)-3-cyclopentyloxy-4-méthoxybenzamide,
N-(méthylthiophényl)-3-cyclopentyloxy-4-méthoxybenzamide,
N-(2-bromophényl)-3-cyclopentyloxy-4-méthoxybenzamide,
N-(2-méthoxycarbonylphényl)-3-cyclopentyloxy-4-méthoxybenzamide
N-(2-aminosulfonylphenyl)-3-cyclopentyloxy-4-méthoxybenzamide,
N-(2-benzoylphényl)-3-cyclopentyloxy-4-méthoxybenzamide,
N-(2-cyanophényl)-3-cyclopentyloxy-4-méthoxybenzamide,
N-(2,5-dichlorophényl)-3-cyclopentyloxy-4-méthoxybenzamide,
N-(2,5-dichlorophényl)-3-cyclopentyloxy-4-méthoxybenzamide,
N-(2-nitrophényl)-3-cyclopentyloxy-4-méthoxybenzamide,
N-(2-diméthylaminophényl)-3-cyclopentyloxy-4-méthoxybenzamide,
N-(2-acétylphényl)-3-cyclopentyloxy-4-méthoxybenzamide,
N-(2-hydroxyphényl)-3-cyclopentyloxy-4-méthoxybenzamide,
N-(2-méthylsulfonylphényl)-3-cyclopentyloxy-4-méthoxybenzamide,
N-(2,6-difluorophényl)-3-cyclopentyloxy-4-méthoxybenzamide,
N-(2,6-difluorophényl)-3-cyclopentyloxy-4-méthoxybenzamide,
N-(2,6-difluorophényl)-3-butoxy-4-méthoxybenzamide,
N-(2,6-difluorophényl)-3-propoxy-4-méthoxybenzamide,
N-(2-chlorophényl)-3-cyclopentyloxy-4-méthoxy(thiobenzamide),
N-(4-chloropyrid-3-yl)-3-cyclopentyloxy-4-méthoxybenzamide,
N-pyrid-2-yl-3-cyclopentyloxy-4-méthoxybenzamide,
N-pyrazin-2-yl-3-cyclopentyloxy-4-méthoxybenzamide,
N-pyrimidin-2-yl-3-cyclopentyloxy-4-méthoxybenzamide,
N-(3-méthylpyrid-2-yl)-3-cyclopentyloxy-4-méthoxybenzamide,
N-pyrid-3-yl-3-cyclopentyloxy-4-méthoxybenzamide,
N-(3-chloropyrid-2-yl)-3-cyclopentyloxy-4-méthoxybenzamide,
N-(3-chloropyrid-4-yl)-3-cyclopentyloxy-4-méthoxybenzamide,
N-pyrid-4-yl-3-cyclopentyloxy-4-méthoxybenzamide,
N-(3,5-dichloropyrid-4-yl)-3-cyclopentyloxy-4-méthoxybenzamide,
N-(3,5-diméthylisoxazol-4-yl)-3-cyclopentyloxy-4-méthoxybenzamide,
N-(4,6-dichloropyrimid-5-yl)-3-cyclopentyloxy-4-méthoxybenzamide,
N-(4-nitrophényl)-3-cyclopentyloxy-4-méthoxybenzamide,
N-(2,3,5,6-tétrafluoropyrid-4-yl)-3-cyclopentyloxy-4-méthoxybenzamide,
N-(3,5-dichloro-2,6-difluoropyrid-4-yl)-3-cyclopentyloxy-4-méthoxybenzamide,
N-(2,4,6-trifluorophényl)-3-cyclopentyloxy-4-méthoxybenzamide,
3,5-dichloro-4-(3-cyclopentyloxy-4-méthoxybenzamido)pyridine-N-oxyde,
N-(3,5-dichloropyrid-4-yl)-3-(exo-8,9,10-trinorbornyl-2-oxy)-4-méthoxybenzamide,
N-(3,5-dichoropyrid-4-yl)-3-cyclopentyloxy-4-méthoxybenzamide,
N-(3,5-dibromopyrid-4-yl)-3-cyclopentyloxy-4-méthoxybenzamide,
N-(3,5-dichloropyrid-4-yl)-3-butoxy-4-méthoxybenzamide,
N-(3-méthyl-5-bromoisothiazol-4-yl)-3-cyclopentyloxy-4-méthoxybenzamide,
N-(3,5-diméthylisothiazol-4-yl)-3-cyclopentyloxy-4-méthoxybenzamide,
N-(3,5-diméthylpyrid-4-yl)-3-cyclopentyloxy-4-méthoxybenzamide,
N-(5-cyano-3-méthylisothiazol-4-yl)-3-cyclopentyloxy-4-méthoxybenzamide,
N-(3,5-dichloropyrid-4-yl)-3-cyclopentyloxy-4-méthoxy(thiobenzamide),
N-(2,6-dichloro-4-méthoxyphényl)-3-cyclopentyloxy-4-méthoxybenzamide,
N-(2,6-dichloro-4-cyanophényl)-3-cyclopentyloxy-4-mèthoxybenzamide,
N-(2,6-dichloro-4-carbamoylphényl)-3-cyclopentyloxy-4-méthoxybenzamide,
N-(2,6-dichloro-4-aminophényl)-3-cyclopentyloxy-4-méthoxybenzamide,
N-(3-chloro-2,5,6-trifluoropyrid-4-yl)-3-cyclopentyloxy-4-méthoxybenzamide,
N-(3,5-dibromopyrid-4-yl)-3-butoxy-4-méthoxybenzamide,
N-(2,6-dichloro-4-méthoxycarbonylphényl)-3-cyclopentyloxy-4-méthoxybenzamide,
N-(4-acétylamino-2,6-dichlorophényl-3-cyclopentyloxy-4-méthoxybenzamide,
N-(3,5-dichloropyrid-4-yl)-3-nonyloxy-4-méthoxybenzamide,
N-(2,6-dichloro-4-formylphényl)-3-cyclopentyloxy-4-méthoxybenzamide,
N-(2,6-dichlorophényl)-3-(exo-8,9,10-trinorbornyl-2-oxy)-4-méthoxybenzamide,
N-(2,3,5-trifluoropyrid-4-yl)-3-cyclopentyloxy-4-méthoxybenzamide,
sel de sodium de N-(3,5-dichloropyrid-4-yl)-3-cyclopentyloxy-4-méthoxybenzamide,
N-(2,6-dichloro-4-éthoxycarbonylphényl)-3-cyclopentyloxy-4-méthoxybenzamide,
N-(2,6-dichloro-4-hydroxymethylphényl)-3-cyclopentyloxy-4-méthoxybenzamide,
N-(3,5-dichloropyrid-4-yl)-3-dodécyloxy-4-méthoxybenzamide,
(R)-N-(3,5-dichloropyrid-4-yl)-3-(exo-8,9,10-trinorbornyl-2-oxy)-4-méthoxybenzamide,
(S)-N-(3,5-dichloropyrid-4-yl)-3-(exo-8,9,10-trinorbornyl-2-oxy)-4-méthoxybenzamide,
N-(2,6-dichloro-4-nitrophényl)--3-cyclopentyloxy-4-méthoxybenzamide,
ou un de leurs sels pharmaceutiquement acceptables.

10. Composé selon la revendication 1, 2, 3, 5, 6, 8 ou 9 qui est le N-(3,5-dichloropyrid-4-yl)-3-cyclopentyloxy-4-méthoxybenzamide.

11. Procédé pour la préparation d'un compose selon l'une des revendications précédentes, qui comprend :
(a) la réaction d'un composé de formule générale (II) : 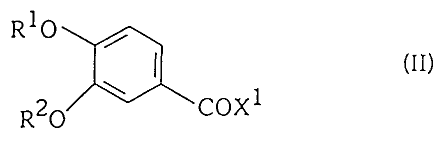 dans laquelle R¹ et R² sont tels que définis dans la revendication 1 et X¹ représente un atome d'halogène, avec un composé de formule générale (III):
NH₂R³ (III)
dans laquelle R³ est tel que défini dans la revendication 1; ou
(b) la réaction d'un composé de formule générale (IV) : 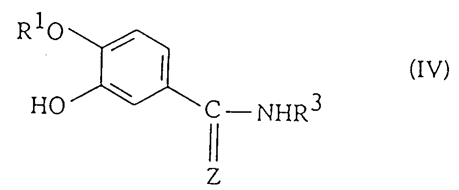 dans laquelle R¹, R² et Z sont tels que définis dans la revendication 1, avec un composé de formule générale (V) :
R²x² (V)
dans laquelle R² est tel que défini dans la revendication 1 et X² représente un atome d'halogène ; éventuellement suivie par la conversion d'un composé de formule générale (I) ainsi obtenu en un autre composé de formule générale (I) ;
et la conversion éventuelle du composé de formule générale (I) ainsi obtenu en un de ses sels.

12. Composition pharmaceutique qui comprend un dérivé de benzamide de formule générale (I) tel que défini dans l'une des revendications 1 à 10, ou un de ses sels pharmaceutiquement acceptables, en association avec un véhicule ou un enrobage pharmaceutiquement acceptable.

13. Utilisation d'un dérivé de benzamide tel que défini dans la revendication 1, pour la préparation d'un médicament utile pour le traitement d'un patient humain ou animal souffrant d'un etat ou sujet à un état qui peut être amélioré par l'administration d'une inhibiteur d'AMP phosphodiéstérase cyclique.
